# EUROPEAN PATENT SPECIFICATION

(11) **EP 3 201 631 B1**
(45) Date of publication and mention of the grant of the patent: **26.05.2021**
(21) Application number: 15771608.5
(22) Date of filing: 01.10.2015
(51) Int. Cl.: G01N 33/68

(54) **HGH DETERMINATION FOR USE TO GUIDE PREVENTION OF A MAJOR ADVERSE CARDIAC EVENT OR A CARDIOVASCULAR DISEASE IN A SUBJECT**
HGH-BESTIMMUNG ZUR VERWENDUNG BEI DER FÜHRUNG DER PRÄVENTION EINES UNERWÜNSCHTEN SCHWEREN HERZEREIGNISSES ODER EINER HERZ-KREISLAUF-ERKRANKUNG BEI EINER PERSON
DÉTERMINATION HGH DESTINÉE À ÊTRE UTILISÉE POUR GUIDER LA PRÉVENTION D'UN ÉVÉNEMENT CARDIAQUE INDÉSIRABLE MAJEUR OU D'UNE MALADIE CARDIOVASCULAIRE CHEZ UN SUJET

(30) Priority: 01.10.2014 EP 14187366
(43) Date of publication of application: 09.08.2017
(73) Proprietor: SphingoTec GmbH, 16761 Hennigsdorf (DE)
(72) Inventor: BERGMANN, Andreas, 13465 Berlin (DE); MELANDER, Olle, 216 11 Limhamn (SE); NG, Leong, Leicester LE2 3NB (GB)
(74) Representative: Kilger, Ute
(86) International application number: PCT/EP2015/072675
(87) International publication number: WO 2016/050904

(56) References cited:
- WO-A1-2010/046136
- WO-A1-2014/108396
- WO-A2-2011/022552
- BANGALORE S ET AL: "Beta-Blockers for Primary Prevention of Heart Failure in Patients With Hypertension", JOURNAL OF THE AMERICAN COLLEGE OF CARDIOLOGY, ELSEVIER, NEW YORK, NY, US, vol. 52, no. 13, 23 September 2008 (2008-09-23), pages 1062-1072, XP025430029, ISSN: 0735-1097, DOI: 10.1016/J.JACC.2008.05.057 [retrieved on 2008-09-16]
- WEBER ET AL: "The Role of the New beta-Blockers in Treating Cardiovascular Disease", AMERICAN JOURNAL OF HYPERTENSION, NATURE PUBLISHING GROUP, UNITED STATES, vol. 18, no. 12, 1 December 2005 (2005-12-01), pages 169-176, XP027816835, ISSN: 0895-7061 [retrieved on 2005-12-01]

### SEQUENCE LISTING

<110> sphingotec GmbH
<120> hGH Determination for Use to Guide Prevention of a Major Adverse Cardiac Event or a Cardiovascular Disease in a Subject
<130> S75037WO
<150> 14187366.1
   <151> 2014-10-01
<160> 4
<170> PatentIn version 3.3
<210> 1
   <211> 217
   <212> PRT
   <213> Homo sapiens
<400> 1
<210> 2
   <211> 202
   <212> PRT
   <213> Homo sapiens
<400> 2
   Met Ala Thr Gly Ser Arg Thr Ser Leu Leu Leu Ala Phe Gly Leu Leu
   1 5 10 15
   Cys Leu Pro Trp Leu Gln Glu Gly Ser Ala Phe Pro Thr Ile Pro Leu
   20 25 30
   Ser Arg Leu Phe Asp Asn Ala Met Leu Arg Ala His Arg Leu His Gln
   35 40 45
   Leu Ala Phe Asp Thr Tyr Gln Glu Phe Asn Pro Gln Thr Ser Leu Cys
   50 55 60
   Phe Ser Glu Ser Ile Pro Thr Pro Ser Asn Arg Glu Glu Thr Gln Gln
   65 70 75 80
   Lys Ser Asn Leu Glu Leu Leu Arg Ile Ser Leu Leu Leu Ile Gln Ser
   85 90 95
   Trp Leu Glu Pro Val Gln Phe Leu Arg Ser Val Phe Ala Asn Ser Leu
   100 105 110
   Val Tyr Gly Ala Ser Asp Ser Asn Val Tyr Asp Leu Leu Lys Asp Leu
   115 120 125
   Glu Glu Gly Ile Gln Thr Leu Met Gly Arg Leu Glu Asp Gly Ser Pro
   130 135 140
   Arg Thr Gly Gln Ile Phe Lys Gln Thr Tyr Ser Lys Phe Asp Thr Asn
   145 150 155 160
   Ser His Asn Asp Asp Ala Leu Leu Lys Asn Tyr Gly Leu Leu Tyr Cys
                   165                 170                 175
   Phe Arg Lys Asp Met Asp Lys Val Glu Thr Phe Leu Arg Ile Val Gln
               180                 185                 190
   Cys Arg Ser Val Glu Gly Ser Cys Gly Phe
           195                 200
<210> 3
   <211> 179
   <212> PRT
   <213> Homo sapiens
<400> 3
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Thr Leu
               100                 105                 110
   Met Gly Arg Leu Glu Asp Gly Ser Pro Arg Thr Gly Gln Ile Phe Lys
           115                 120                 125
   Gln Thr Tyr Ser Lys Phe Asp Thr Asn Ser His Asn Asp Asp Ala Leu
       130                 135                 140
   Leu Lys Asn Tyr Gly Leu Leu Tyr Cys Phe Arg Lys Asp Met Asp Lys
   145                 150                 155                 160
   Val Glu Thr Phe Leu Arg Ile Val Gln Cys Arg Ser Val Glu Gly Ser
                   165                 170                 175
   Cys Gly Phe
<210> 4
   <211> 171
   <212> PRT
   <213> Homo sapiens
<400> 4
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Ile Phe Lys Gln Thr Tyr Ser Lys Phe Asp Thr
           115                 120                 125
   Asn Ser His Asn Asp Asp Ala Leu Leu Lys Asn Tyr Gly Leu Leu Tyr
       130                 135                 140
   Cys Phe Arg Lys Asp Met Asp Lys Val Glu Thr Phe Leu Arg Ile Val
   145                 150                 155                 160
   Gln Cys Arg Ser Val Glu Gly Ser Cys Gly Phe
                   165                 170

## Description

The present invention describes a method of determining whether a subject has a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker)) or HMG-CoA reductase inhibitors (statins) comprising the steps:
- determining the level of hGH, and/or its isoforms in a blood sample of said subject and
- comparing the determined level of hGH and/or its isoforms in said blood sample with a pre-determined threshold and
- wherein in case the determined level of hGH and/or its isoforms is above said pre-determined threshold then the subject is identified as having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) and
- wherein in case the determined level of hGH and/or its isoforms is below said pre-determined threshold then the subject is identified as not having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins).

The use of biomarkers for stratification of patients suffering from or being at risk to develop cardiovascular disease for a successful pharmacologic intervention is principally known. However, this has been restricted so far to biomarkers associated with vasoactive or inflammatory activities, e.g. in WO/2010/128071, in Richards AM, et al. Australia-New Zealand Heart Failure Group. Journal of the American College of Cardiology 2001, 37(7):1781-1787 and in Ridker et al: The New England journal of medicine 2008, 359(21):2195-2207.

Human growth hormone (hGH) has never been described in this context. It is only known that relatively elevated levels of plasma hGH in the normal population (especially in males) are associated with an increased risk for the development of incident adverse cardiac events in WO/2014/108396.

Here we describe that elevated levels of plasma hGH and/or its isoforms in subjects are associated with the success of therapy with hypertensive drugs (e.g. Angiotensin converting enzyme inhibitor (ACE inhibitor), Angiotensin Receptor Blocker (ARB), Beta-Adrenoreceptor Blocker (Beta-blocker) and/or statins), whereas such therapy is not efficacious in patients with relatively low levels of plasma hGH and/or its isoforms. Thus, measurement of hGH and/or its isoforms in subjects is suitable to stratify patients for pharmacological therapy.

Subject matter of the present invention is a method of determining whether a subject has a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) comprising the steps:
- determining the level of hGH and/or its isoforms selected from the group comprising hGH isoform 1, hGH isoform 2, hGH isoform 3 and hGH isoform 4 in a blood sample of said subject and
- comparing the determined level of hGH and/or said isoforms thereof in said blood sample with a pre-determined threshold and
- wherein in case the determined level of hGH and/or its isoforms is above said pre-determined threshold then the subject is identified as having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) and
- wherein in case the determined level of hGH and/or its isoforms is below said pre-determined threshold then the subject is identified as not having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins).

In one specific embodiment said subject does not have hypertension, wherein having hypertension is defined as having blood pressure of at least >140mmHG (systolic) to 90 mmHG (diastolic).

In one specific embodiment said subject has not yet been treated with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins).

In one embodiment reducing the vascular risk by lowering blood pressure in a subject means preventing a major cardiovascular event and/or a cardiovascular disease in said subject.

In one specific embodiment of the method said subject has suffered an acute myocardial infarction or acute heart failure within the last 2 weeks, preferably within the last one week, preferably within the last 36 hours.

In one specific embodiment of the method according to the invention said subject has never had a major cardiovascular event and has never had any cardiovascular disease. In another specific embodiment of the method according to the invention said subject has carotid plaque but no symptoms of carotid artery disease. An established method to detect the presence of an atherosclerotic disease and to monitor its regression, arrest or progression is the measurement of the intima-media thickness (IMT) (de Groot et al., Nature Reviews Cardiology 5, 280-288 (May 2008) | doi:10.1038/ncpcardio1163). This is a measurement of the thickness of tunica intima and tunica media, the innermost two layers of the wall of an artery. The measurement is usually made by external ultrasound and occasionally by internal, invasive ultrasound catheters. The FDA has approved IMT as a surrogate marker of atherosclerotic disease for application in clinical trials. The extent of IMT has been associated with cardiovascular outcome and its change of over time (statistically significant of IMT per year) with efficacy of drugs (de Groot et al., Nature Reviews Cardiology 5, 280-288 (May 2008) | doi:10.1038/ncpcardio1163; Hedblad et al., Circulation. 2001;103:1721-1726.)

Said major cardiovascular event or cardiovascular disease may be selected from the group comprising heart failure, artherosclerosis, hypertension, cardiomyopathy, myocardial infarction and stroke.

In one embodiment of the invention said subject is male.

In one embodiment of the method according to the invention said method is used for prevention of a major cardiovascular event or prevention of a cardiovascular disease.

Said major cardiovascular event may be selected from the group comprising myocardial infarction, acute heart failure, stroke and cardiovascular death.

The blood sample may be selected from the group comprising whole blood, serum and plasma.

In one embodiment of the invention the blood sample is a fasting sample.

Fasting sample means a sample, which has been drawn from a subject, which has not consumed any food or solubilized nutrients for at least 8 hours prior to blood sampling. In a specific embodiment, such fasting sample is taken from a subject between 7:00 and 11:30 a.m., after the subject has fastened at least 8 hours.
In another embodiment fasting levels of growth hormone (hGH) means the level of growth hormone (hGH) determined in blood, serum or plasma of fasting subjects that did not have food uptake 12 h prior sample taking. In said embodiment fasting subject means a subject that had 12 h prior sample taking no food up-take.

The term "subject" as used herein refers to a living human or non-human organism. Preferably herein the subject is a human subject. The subject may be healthy or diseased if not stated otherwise. The term "elevated level" means a level above a certain threshold level.

In one specific embodiment of the invention the measurement method or assay that is used to determine the level of hGH and/or its isoforms has an analytical sensitivity, which is sufficient to quantify the lower 95^{th} percentile of the normal range from fasting healthy, preferably at least 40 years old males with an interassay CV, which does not exceed 20%. An example for such assay is described in WO/2014/108396.

Assay sensitivity may be defined by the "analytical" assay sensitivity that is defined as hGH concentration (standard curve readout) at a median signal of e.g. 20 determinations of hGH depleted sample + 2 standard deviations (SD).

Alternatively, the assay sensitivity may be defined by the "functional" assay sensitivity, which reflects more the limit of detection in a clinical routine. The functional assay sensitivity is defined as determination of the inter assay coefficient of variation (CV) of plasma (patient samples) in a variety of individual samples with different hGH concentrations. The functional assay sensitivity is the lowest hGH concentration with an inter assay CV of less than 20%.

Assay sensitivities may depend upon the used method of calibration and the used hGH assay. Different hGH assays may detect different sets of hGH isoforms, leading to different quantitative results meaning leading to different thresholds and sensitivity needs depending on the assay and the calibration method used.
To overcome this possible heterogeneity of different hGH assays, assay calibrations may be corrected by correlating plasma hGH values of individual subjects. If the slope of the correlation curve is different from 1, the hGH values (the calibration) can be corrected by the differential factor leading to a comparable calibration, comparable sensitivity analysis and comparable thresholds.
The above correction may be performed in order to compare the results obtained by different assays.
The assay described in example 1 predominantly recognizes hGH isoform 1 and is calibrated by the recombinant Human Growth Hormone (NIBSC code 98/574, National Institute for Biological Standards and Control, Herfordshire, UK).
In a specific method according to the invention said fasting level of growth hormone (hGH) and/or its isoforms in a bodily fluid is determined by an ultrasensitive assay having an analytical assay sensitivity (that is defined as hGH concentration at a median signal of 20 determinations of hGH depleted sample + 2 standard deviations (SD)) of less than 100 pg/ml, preferred less than 50 pg/ml, preferred less than 30 pg/ml, preferred less than 20 pg/ml, preferred less than 10 pg/ml, preferred less than 5 pg/ml, preferred 2 pg/ml.
Alternatively, the ultrasensitive assay having a functional assay sensitivity (see above) of less than 400 pg/ml, preferred less than 200 pg/ml, preferred less than 120 pg/ml, preferred less than 80 pg/ml, preferred less than 40 pg/ml, preferred less than 20 pg/ml, preferred 10 pg/ml most preferred less than 8.5 pg/ml.

The assay can be an immunoassay or another detection method, for instance mass spectrometry.

For immunization and for calibration we used recombinant Human Growth Hormone (NIBSC code 98/574, National Institute for Biological Standards and Control, Herfordshire, UK).

In one embodiment of the invention said assay is conducted as described in Example 1.

Said assay may be selective for one specific hGH isoform or maybe specific for and determines more than one or all secreted isomers of hGH selected from the group comprising hGH isoform 1, isoform 2, isoform 3, and isoform 4 (see SEQ ID NO. 1-4) in said bodily fluid.

Isoforms of growth hormone (hGH) may be selected from the group comprising hGH isoform 1 (22KD), hGH isoform 2, hGH isoform 3, and hGH isoform 4.

### Sequences of the isoforms:

In one specific embodiment of the invention the measurement method that is used to determine the level of hGH and/or its isoforms selected from the group comprising hGH isoform 1, hGH isoform 2, hGH isoform 3 and hGH isoform 4 is an assay having an assay sensitivity of at least 50 pg/ml.

In one specific embodiment of the invention the measurement method that is used to determine the level of hGH and/or its isoforms selected from the group comprising hGH isoform 1, hGH isoform 2, hGH isoform 3 and hGH isoform 4 is an immuno assay having an assay sensitivity of at least 50 pg/ml.

In one embodiment of the invention the pre-determined threshold is from above 330 pg/ml. In one embodiment of the invention the pre-determined threshold is from above 330 pg/ml in a subject that has suffered an acute myocardial infarction within the last 2 weeks, preferably within the last one week, preferably within the last 36 hours.

In one embodiment the threshold may be pre-determined as follows:
- Comparison of concentration of hGH and/or its isoforms selected from the group comprising hGH isoform 1, hGH isoform 2, hGH isoform 3 and hGH isoform 4 in a bodily fluid obtained from said subject with the median of the levels of hGH and/or said isoforms thereof in a bodily fluid obtained from an ensemble of pre-determined samples in a randomly selected population of subjects having comparable baseline conditions as said subject,
- Comparison of concentration of hGH and/or its isoforms selected from the group comprising hGH isoform 1, hGH isoform 2, hGH isoform 3 and hGH isoform 4 in a bodily fluid obtained from said subject with a quantile of the levels of hGH and/or said isoforms thereof in a bodily fluid obtained from an ensemble of pre-determined samples in a population of subjects having comparable baseline conditions as said subject,
- Calculation based on Cox Proportional Hazards analysis or by using Risk index calculations such as the NRI (Net Reclassification Index) or the IDI (Integrated Discrimination Index).

The person skilled in the art understands that a pre-determined threshold has to be seen in the context of assay used, calibration methods used and other factors.

Subject matter according to the present invention is a method wherein the level, preferably fasting level, of growth hormone (hGH) and/or its isoforms selected from the group comprising hGH isoform 1, hGH isoform 2, hGH isoform 3 and hGH isoform 4 is determined by using a binder to growth hormone (hGH) and/or said isoforms thereof.

In one embodiment of the invention said binder is selected from the group comprising an antibody, an antibody fragment or a non-Ig-Scaffold binding to growth hormone (hGH) and/or its isoforms selected from the group comprising hGH isoform 1, hGH isoform 2, hGH isoform 3 and hGH isoform 4.

In one specific embodiment of the invention an immunoassay is used that comprises at least two antibodies that bind to hGH and/or its isoforms selected from the group comprising hGH isoform 1, hGH isoform 2, hGH isoform 3 and hGH isoform 4.

According to the invention the diagnostic binder is selected from the group consisting of antibodies e.g. IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains, e.g. Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines.

In a preferred embodiment each of the at least two antibodies that bind to hGH and/or its isoforms selected from the group comprising hGH isoform 1, hGH isoform 2, hGH isoform 3 and hGH isoform 4 have an affinity towards hGH and/or said isoforms thereof of at least 10⁸ M⁻¹, preferred affinity constant is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention. Binding affinity may be determined using the Biacore method, offered as service analysis e.g. at Biaffin, Kassel, Germany (http://www.biaffin.com/de/).

To determine the affinity of the antibodies, the kinetics of binding of hGH to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare) (Lorenz et al.," Functional Antibodies Targeting IsaA of Staphylococcus aureus Augment Host Immune Response and Open New Perspectives for Antibacterial Therapy"; Antimicrob Agents Chemother. 2011 January; 55(1): 165-173).

In addition to antibodies other biopolymer scaffolds are well known in the art to complex a target molecule and have been used for the generation of highly target specific biopolymers. Examples are aptamers, spiegelmers, anticalins and conotoxins. Non-Ig scaffolds may be protein scaffolds and may be used as antibody mimics as they are capable to bind to ligands or antigenes. Non-Ig scaffolds may be selected from the group comprising tetranectin-based non-Ig scaffolds *(e.g.* described in US 2010/0028995), fibronectin scaffolds *(e.g.* described in EP 1266 025; lipocalin-based scaffolds (e.g. described in WO 2011/154420); ubiquitin scaffolds *(e.g.* described in WO 2011/073214), transferring scaffolds *(e.g.* described in US 2004/0023334), protein A scaffolds (e.g. described in EP 2231860), ankyrin repeat based scaffolds (e.g. described in WO 2010/060748), microproteins preferably microproteins forming a cystine knot) scaffolds (e.g. described in EP 2314308), Fyn SH3 domain based scaffolds *(e.g.* described in WO 2011/023685) EGFR-A-domain based scaffolds *(e.g.* described in WO 2005/040229) and Kunitz domain based scaffolds (e.g. described in EP 1941867).

In one embodiment of the invention it may be a so-called POC-test (point-of-care), that is a test technology that allows performing the test within less than 1 hour near the patient without the requirement of a fully automated assay system. One example for this technology is the immunochromatographic test technology.

In one embodiment of the invention such an assay is a sandwich immunoassay using any kind of detection technology including but not restricted to enzyme label, chemiluminescence label, electrochemiluminescence label, preferably a fully automated assay. In one embodiment of the invention such an assay is an enzyme labeled sandwich assay. Examples of automated or fully automated assay comprise assays that may be used for one of the following systems: Roche Elecsys®, Abbott Architect®, Siemens Centauer®, Brahms Kryptor®, Biomerieux Vidas®, Alere Triage®.

A variety of immunoassays are known and may be used for the assays and methods of the present invention, these include: radioimmunoassays ("RIA"), homogeneous enzymemultiplied immunoassays ("EMIT"), enzyme linked immunoadsorbent assays ("ELISA"), apoenzyme reactivation immunoassay ("ARIS"), dipstick immunoassays and immunochromatography assays.

In one embodiment of the invention at least one of said two binders is labeled in order to be detected.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

In a preferred embodiment said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person (Ludvigsson J, Andersson E, Ekbom A, Feychting M, Kim J-L, Reuterwall C, et al. External review and validation of the Swedish national inpatient register. BMC Public Health. 2011;11(1):450).

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in (D'Agostino RB, Sr., Vasan RS, Pencina MJ, Wolf PA, Cobain M, Massaro JM, et al. General cardiovascular risk profile for use in primary care: the Framingham Heart Study. Circulation. 2008;117(6):743-53. Epub 2008/01/24). Preferred chemiluminescent dyes are acridiniumesters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably more than 10⁸ M⁻¹.

In the context of the present invention, "binder molecules" are molecules which may be used to bind target molecules or molecules of interest, *i.e.* analytes *(i.e.* in the context of the present invention hGH and/or isomers thereof), from a sample. Binder molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, binder molecules may for instance be selected from the group comprising a nucleic acid molecule, a carbohydrate molecule, a PNA molecule, a protein, an antibody, a peptide or a glycoprotein. Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

Chemiluminescent label may be acridinium ester label, steroid labels involving isoluminol labels and the like.

Enzyme labels may be lactate dehydrogenase (LDH), creatine kinase (CPK), alkaline phosphatase, aspartate aminotransferase (AST), alanine aminotransferase (ALT), acid phosphatase, glucose-6-phosphate dehydrogenase and so on.

In one embodiment of the invention at least one of said two binders is bound to a solid phase as magnetic particles, and polystyrene surfaces.

In one embodiment of the assays for determining the level of growth hormone (hGH), and/or its isoforms in a sample according to the present invention such assay is a sandwich assay, preferably a fully automated assay. It may be an ELISA fully automated or manual. It may be a so-called POC-test (point-of-care). Examples of automated or fully automated assay comprise assays that may be used for one of the following systems: Roche Elecsys®, Abbott Architect®, Siemens Centauer®, Brahms Kryptor®, Biomerieux Vidas®, Alere Triage®. Examples of test formats are provided above.

In one embodiment of the assays for determining the level of growth hormone (hGH) and/or its isoforms in a sample according to the present invention at least one of said two binders is labeled in order to be detected. Examples of labels are provided above.

In one embodiment of the assays for determining the level of growth hormone (hGH) and/or its isoforms selected from the group comprising hGH isoform 1, hGH isoform 2, hGH isoform 3 and hGH isoform 4 in a sample according to the present invention at least one of said two binders is bound to a solid phase. Examples of solid phases are provided above.

In one embodiment of the assays for determining the level of growth hormone (hGH) and/or its isoforms selected from the group comprising hGH isoform 1, hGH isoform 2, hGH isoform 3 and hGH isoform 4 in a sample according to the present invention said label is selected from the group comprising chemiluminescent label, enzyme label, fluorescence label, radioiodine label.
A further subject of the present invention is a kit comprising an assay according to the present invention wherein the components of said assay may be comprised in one or more container.

In one embodiment of the invention the level of hGH and/or its isoforms selected from the group comprising hGH isoform 1, hGH isoform 2, hGH isoform 3 and hGH isoform 4 is determined by mass spectrometry.

In one specific embodiment said method according to the present invention is used for monitoring the vascular risk of said subject. Thus, subject matter of the present invention is also a method of determining at least twice, preferably annually, whether a subject has a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) comprising the steps:
- determining the level of hGH and/or its isoforms selected from the group comprising hGH isoform 1, hGH isoform 2, hGH isoform 3 and hGH isoform 4 in a blood sample of said subject and
- comparing the determined level of hGH and/or said isoforms thereof in said blood sample with a pre-determined threshold and
- wherein in case the determined level of hGH and/or said isoforms thereof is above said pre-determined threshold then the subject is identified as having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) and
- wherein in case the determined level of hGH and/or said isoforms thereof is below said pre-determined threshold then the subject is identified as not having a vascular risk that can be reduced by blood pressure lowering therapy with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins).

In said monitoring method the above mentioned method steps are conducted at least once a year.

The method according to the present invention may in one embodiment of the invention further comprise:
- recommendation of administration of antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) to a subject whose determined level of hGH and/or its isoforms, is above said pre-determined threshold and who is identified as having a vascular risk that can be reduced by blood pressure lowering therapy with antihypertensive drugs or
- recommendation of withholding with antihypertensive drugs (e.g. Angiotensin converting enzyme inhibitor (ACE inhibitor), Angiotensin Receptor Blocker (ARB), Beta-Adrenoreceptor Blocker (Beta-blocker) and/or statins) to a subject whose determined level of hGH and/or its isoforms is below said pre-determined threshold and who is identified as not having a vascular risk that can be reduced by blood pressure lowering therapy with antihypertensive drugs.

In one embodiment of the invention the hypertensive drug is selected from the group comprising Angiotensin converting enzyme inhibitor (ACE inhibitor), Angiotensin Receptor Blocker (ARB), and Beta-Adrenoreceptor Blocker (Beta-blocker), statins and a combination thereof as well as a combination with other drugs, such as for instance diuretics.

In one embodiment of the invention the Angiotensin converting enzyme inhibitor (ACE inhibitor) is selected from the group comprising Benazepril, Captopril, Cilazapril, Enalapril, Fosinopril, Imidapril, Lisinopril, Moexipril, Perindopril, Quinapril, Ramipril, Spirapril, Trandolapril, and Zofenopril.

In one embodiment of the invention the Angiotensin Receptor Blocker (ARB) is selected from the group comprising Azilsartan, Azilsartanmedoxomil, Candesartan, Candesartancilexetil, Eprosartan, Fimasartan, Irbesartan, Losartan, Milfasartan, Olmesartan, Pomisartan, Pratosartan, Ripisartan, Saprisartan, Tasosartan, Telmisartan, and Valsartan.

In one embodiment of the invention the Beta-Adrenoreceptor Blocker (Beta-blocker) is selected from the group comprising Atenolol, Bisoprolol, Metoprolol, Nebivolol, Esmolol, Betaxolol, Acebutolol, Celiprolol, Propranolol, Bupranolol, Timolol, Carvedilol, Sotalol, Nadolol, Pindolol, Oxprenolol, Alprenolol and Carteolol

In one embodiment of the invention the statin is selected from the group comprising Atorvastatin, Fluvastatin, Lovastatin, Pitavastatin, Pravastatin, Rosuvastatin, Simvastatin, Cerivastatin and Mervastatin (Compactin) and a combination thereof as well as a combination with other drugs.

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs .

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins) for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs and wherein said subject does not have hypertension.

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins) for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs and wherein said subject does not have hypertension and wherein said Angiotensin converting enzyme inhibitor (ACE inhibitor) is selected from the group comprising Benazepril, Captopril, Cilazapril, Enalapril, Fosinopril, Imidapril, Lisinopril, Moexipril, Perindopril, Quinapril, Ramipril, Spirapril, Trandolapril, and Zofenopril.

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins) for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs and wherein said subject does not have hypertension and wherein said Angiotensin Receptor Blocker (ARB) is selected from the group comprising Azilsartan, Azilsartanmedoxomil, Candesartan, Candesartancilexetil, Eprosartan, Fimasartan, Irbesartan, Losartan, Milfasartan, Olmesartan, Pomisartan, Pratosartan, Ripisartan, Saprisartan, Tasosartan, Telmisartan, and Valsartan.

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins) for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs and wherein said subject does not have hypertension and wherein said statin is selected from the group comprising: Atorvastatin, Fluvastatin, Lovastatin, Pitavastatin, Pravastatin, Rosuvastatin, Simvastatin, Cerivastatin and Mervastatin (Compactin).

Subject matter of the present invention is an antihypertensive drug (e.g. Angiotensin converting enzyme inhibitor (ACE inhibitor), Angiotensin Receptor Blocker (ARB), Beta-Adrenoreceptor Blocker (Beta-blocker) and/or statins) for use in preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of the present invention as having a vascular risk that can be reduced by blood pressure lowering therapy with antihypertensive drugs and wherein said subject does not have hypertension and wherein said Beta-Adrenoreceptor Blocker (Beta-blocker) is selected from the group comprising Atenolol, Bisoprolol, Metoprolol, Nebivolol, Esmolol, Betaxolol, Acebutolol, Celiprolol, Propranolol, Bupranolol, Timolol, Carvedilol, Sotalol, Nadolol, Pindolol, Oxprenolol, Alprenolol and Carteolol.

Most of the above active compounds may be formulated in different salt forms. Pharmaceutically acceptable salts may be obtained while using the common procedures. Thus, subject matter are also all pharmaceutically acceptable salts of the above mentioned active compounds.
Such salt forms may be common alkaline and acid addition salts obtained from conversion with respective organic or inorganic acids. The acids which form salts of the active compounds include sulfuric acid, sulfonic acid, phosphoric acid, nitrous acid, nitric acid, perchloric acid, hydrobromic acid, hydrochloric acid, formic acid, acetic acid, propionic acid, succinic acid, oxalic acid, glucuronic acid (in the left and dextrorotatory form), lactic acid, malic acid, tartaric acid, (hydroxymalonic, hydroxypropanoicdicarbonic acid), fumaric acid, citric acid, ascorbic acid, maleic acid, malonic acid, hydroxymaleic acid, pyruvic acid, phenylacetic acid (o-, m-, p-) toluic acid, benzoic acid, p-aminobenzoic acid, salicylic, p-amino-salicylic acid, methylsulfonic-, ethylsulfonic-, hydroxy methylsulfonic acid, ethylenesulfonic acid, p-toluenesulfonic, naphthalenesulfonic, naphthylaminosulfonic acid, sulfanilic acid, camphorsulfonic acid, quinic acid, o-methyl mandelic acid, picric acid (2,4,6-trinitrophenol), adipic acid, amino acids such as methionine, tryptophane, arginine, and especially acidic amino acids such as glutamine or aspartic acid.
In case acid substituents are present in said active compounds also basic addition salts may be formed, particularly with alkali metals, as well as with amino acids. Therefore, alkali metal salts such as the sodium, potassium, lithium salt or the magnesium, calcium salt, alkylamino salts or salts formed with amino acids, for example with amino acids such as lysine alkaline. The preferred specific salts in accordance with the invention are selected from a group comprising sulphate, bisulphate, nitrate, phosphate, hydrogen phosphate, hydrochloride, hydrobromide, hydroiodide, acetate, tartrate, lactate, citrate, gluconate, fumarate, maleate, hydroxyl maleate, succinate, pamoate, benzoate, propionate, pyruvate, oxalate, malonate, cinnamate, salicylate, alkyl sulphonate, aryl sulphonate, and aralkyl sulphonate.

Suitable salts of the active compounds are all acid addition salts or all salts with bases. Particular mention may be made of the pharmacologically tolerable inorganic and organic acids and bases customarily used in pharmacy.
Those suitable are, on the one hand, water insoluble and, particularly, water soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid such as (-)-L-malic acid or (+)-D-malic acid, fumaric acid, succinic acid, oxalic acid, tartaric acid such as (+)-L-tartaric acid or (-)-D-tartaric acid or meso-tartaric acid, embonic acid, stearic acid, toluenesulphonic acid, methanesulphonic acid or 3-hydroxy-2-naphthoicacid, the acids being employed in salt preparation - depending on whether a mono- or polybasic acid is concerned and depending on which salt is desired - in an equimolar quantitative ratio or one differing therefrom. Further, glutamate and aspartate are suitable salts of the above mentioned active compounds.
Pharmacologically intolerable salts, which can be obtained, for example, as process products or by-products during the preparation of the compounds according to the present invention on an industrial scale, are converted into pharmacologically tolerable salts by processes known to the person skilled in the art.
The term "pharmaceutical acceptable" in the context of the invention means that the relevant derivatives, functional equivalents, salts, solvates, hydrates, excipients, carrier, diluents, and solvents according to the invention are safe and effective for the comprised use in mammals and that possess the desired biological activity and/or function, respectively.

Some selected embodiments of the inventions are listed below:
1) Method of determining whether a subject has a vascular risk that can be reduced by blood pressure lowering therapy with antihypertensive drugs comprising the steps:
   - determining the level of hGH and/or its isoforms selected from the group comprising hGH isoform 1, hGH isoform 2, hGH isoform 3 and hGH isoform 4 in a blood sample of said subject and
   - comparing the determined level of hGH and/or said isoforms thereof in said blood sample with a pre-determined threshold and
   - wherein in case the determined level of hGH and/or said isoforms thereof is above said pre-determined threshold then the subject is identified as having a vascular risk that can be reduced by blood pressure lowering therapy with antihypertensive drugs and
   - wherein in case the determined level of hGH and/or said isoforms thereof is below said pre-determined threshold then the subject is identified as not having a vascular risk that can be reduced by blood pressure lowering therapy with antihypertensive drugs.
2) Method of determining whether a subject has a vascular risk that can be reduced by blood pressure lowering therapy with antihypertensive drugs according to embodiment 1, wherein said subject does not have hypertension, and wherein having hypertension is defined as having blood pressure of at least > 140mm HG (systolic) to 90 mmHG (diastolic).
3) Method of determining whether a subject has a vascular risk that can be reduced by blood pressure lowering therapy with antihypertensive drugs according to embodiment 1 or 2, wherein the blood sample is taken from a subject that has not yet been treated with antihypertensive drugs.
4) Method of determining whether a subject has a vascular risk that can be reduced by blood pressure lowering therapy with antihypertensive drugs according to embodiment 1, 2 or 3, wherein reducing the vascular risk by lowering blood pressure in a subject means preventing a major cardiovascular event and/or a cardiovascular disease in said subj ect.
5) Method of determining whether a subject has a vascular risk that can be reduced by blood pressure lowering therapy with antihypertensive drugs according to any of embodiments 1 to 4, wherein said subject has never had a major cardiovascular event and has never had any cardiovascular disease.
6) Method of determining whether a subject has a vascular risk that can be reduced by blood pressure lowering therapy with antihypertensive drugs according to any of the embodiments 1 to 4, wherein said subject has suffered an acute myocardial infarction or acute heart failure within the last 2 weeks, preferably within the last one week, preferably within the last 36 hours.
7) Method of determining whether a subject has a vascular risk that can be reduced by blood pressure lowering therapy with antihypertensive drugs according to any of the embodiments 1 to 6, wherein said antihypertensive drug is selected from the group comprising Angiotensin converting enzyme inhibitor (ACE inhibitor), Angiotensin Receptor Blocker (ARB), and Beta-Adrenoreceptor Blocker (Beta-blocker), statin or combinations thereof.
8) Method of determining whether a subject has a vascular risk that can be reduced by blood pressure lowering therapy with antihypertensive drugs according to any of the preceding embodiments, wherein the blood sample is selected from the group comprising whole blood, serum, and plasma.
9) Method of determining whether a subject has a vascular risk that can be reduced by blood pressure lowering therapy with antihypertensive drugs according to any of the preceding embodiments, wherein the blood sample is a fasting sample.
10) Method of determining whether a subject has a vascular risk that can be reduced by blood pressure lowering therapy with antihypertensive drugs according to any of the preceding embodiments, wherein the measurement method that is used to determine the level of hGH and/or its isoforms selected from the group comprising hGH isoform 1, hGH isoform 2, hGH isoform 3 and hGH isoform 4 has a sensitivity of at least 50 pg/ml.
11) Method of determining whether a subject has a vascular risk that can be reduced by blood pressure lowering therapy with antihypertensive drugs according to any of the preceding embodiments, wherein the measurement method that is used to determine the level of hGH and/or its isoforms selected from the group comprising hGH isoform 1, hGH isoform 2, hGH isoform 3 and hGH isoform 4 is an immunoassay having an assay sensitivity of at least 50 pg/ml.
12) Method of determining whether a subject has a vascular risk that can be reduced by blood pressure lowering therapy with antihypertensive drugs according to any of the preceding embodiments, wherein the pre-determined threshold is 330 pg/ml or above.
13) Method of determining whether a subject has a vascular risk that can be reduced by blood pressure lowering therapy with antihypertensive drugs according any of the embodiments 1-12, wherein the level of hGH and/or its isoforms selected from the group comprising hGH isoform 1, hGH isoform 2, hGH isoform 3 and hGH isoform 4 is determined by an immunoassay that comprises at least two antibodies that bind to hGH and/or its isoforms selected from the group comprising hGH isoform 1, hGH isoform 2, hGH isoform 3 and hGH isoform 4.
14) Method of determining whether a subject has a vascular risk that can be reduced by blood pressure lowering therapy with antihypertensive drugs according to embodiment 13, wherein each of the at least two antibodies that bind to hGH and/or its isoforms selected from the group comprising hGH isoform 1, hGH isoform 2, hGH isoform 3 and hGH isoform 4 have an affinity towards hGH and/or said isoforms thereof that is at least 10⁸M⁻¹.
15) Method of determining whether a subject has a vascular risk that can be reduced by blood pressure lowering therapy with antihypertensive drugs according to any of the preceding embodiments, wherein said method is used for prevention of a major cardiovascular event and/or a cardiovascular disease in said subject selected from the group comprising heart failure, atherosclerosis, hypertension, cardiomyopathy, myocardial infarction and stroke.
16) Method of determining whether a subject has a vascular risk that can be reduced by blood pressure lowering therapy with antihypertensive drugs according to any of the preceding embodiments, wherein the method further comprises:
   - recommendation of administration of antihypertensive drugs to a subject whose determined level of hGH and/or its isoforms is above said pre-determined threshold and who is identified as having a vascular risk that can be reduced by blood pressure lowering therapy with antihypertensive drugs or
   - recommendation of withholding with antihypertensive drugs to a subject whose determined level of hGH and/or its isoforms is below said pre-determined threshold and who is identified as not having a vascular risk that can be reduced by blood pressure lowering therapy with antihypertensive drugs.
17) An antihypertensive drug for use in a method of preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of any of embodiments 1-16 as having a vascular risk that can be reduced by blood pressure lowering therapy with antihypertensive drugs, wherein said cardiovascular event is selected from the group comprising myocardial infarction, acute heart failure, stroke and cardiovascular death.
18) An antihypertensive drug for use in a method of preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of any of embodiments 1-16 as having a vascular risk that can be reduced by blood pressure lowering therapy with antihypertensive drugs, wherein said subject does not have hypertension.
19) A method for monitoring the therapy response of a subject at vascular risk that is treated with antihypertensive drugs comprising the steps:
   - determining the level of hGH and/or its isoforms selected from the group comprising hGH isoform 1, hGH isoform 2, hGH isoform 3 and hGH isoform 4 in a blood sample of said subject taken at a time point XI with antihypertensive drugs and
   - determining the level of hGH and/or said isoforms thereof in a blood sample of said subject taken at a time point X2 with antihypertensive drugs, and
   - wherein time point XI is either before the beginning of therapy or after starting the therapy but in any event earlier than time point X2, and
   - wherein time point X2 is after starting the therapy and in any event later than time point XI
   - comparing the determined level of hGH and/or said isoforms thereof in said blood sample taken at point XI with the determined level of hGH and/or said isoforms thereof in said blood sample taken at time point X2
   - wherein a lower level of hGH and/or said isoforms thereof in said blood sample taken at time point X2 compared to the level of hGH and/or said isoforms thereof in said blood sample taken at time point XI indicates a therapy response.
20) A method for monitoring the therapy response of a subject at vascular risk that is treated with antihypertensive drugs according to embodiment 19, wherein the determined level of hGH and/or its isoforms selected from the group comprising hGH isoform 1, hGH isoform 2, hGH isoform 3 and hGH isoform 4 in said blood sample taken at point XI is above a pre-determined threshold and the subject has been identified as having a vascular risk that can be reduced by blood pressure lowering therapy with antihypertensive drugs.
21) A method for monitoring the therapy response of a subject at vascular risk that is treated with antihypertensive drugs according to embodiment 19 or 20, wherein the time point XI is before starting the therapy.
22) A method for monitoring the therapy response of a subject at vascular risk that is treated with antihypertensive drugs according to any of embodiments 19 to 21, wherein time point X2 is 12 months, 24 months, or 36 months after time point XI.
23) A method according to any of embodiments 1, 16, 19 - 22, or an antihypertensive drug for use in a method according to any of embodiments 17 and 18, wherein the antihypertensive drugs are selected from the group comprising Angiotensin converting enzyme inhibitor (ACE inhibitor), Angiotensin Receptor Blocker (ARB), Beta-Adrenoreceptor Blocker (Beta-blocker), statin, or combinations thereof.

### Examples

### Example 1, us-hGH Assay

Plasma samples were measured using a hGH assay as described previously in WO/2014/108396. The measurement was made with an ultra-sensitivity chemiluminescence immunoassay (see example 1 us-hGH assay). The detection limit was 2 pg/ml, functional assay sensitivity was 8.5 pg/ml.

### Chemicals

If not stated otherwise, chemicals were obtained at p.a. grade from Merck (Darmstadt, Germany).

### Antigen

For immunization and for calibration we used recombinant Human Growth Hormone (NIBSC code 98/574, National Institute for Biological Standards and Control, Herfordshire, UK).

### Development of Antibodies

Mouse monoclonal antibodies against hGH were developed by UNICUS (Karlsburg, Germany).

### The antibodies were generated according to the following method:

A BALB/c mouse were immunized with 100 µg hGH at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intra venous injection.

Splenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20 % fetal calf serum and HAT-supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined. (Lane, R.D. "A short-duration polyethylene glycol fusiontechnique for increasing production of monoclonal antibody-secreting hybridomas", J. Immunol. Meth. 81: 223-228; (1985) - Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996))

### Monoclonal antibody production

We selected 5 antibodies for further investigations.

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Labelling and coating of antibodies.

All antibodies were labelled with acridinium ester according the following procedure:
Labelled compound (tracer): 100 µg (100 µl) antibody (1 mg/ml in PBS, pH 7.4), was mixed with 10 µl Acridinium NHS-ester (1 mg/ml in acetonitrile, InVent GmbH, Germany) (EP 0353971) and incubated for 20 min at room temperature. Labelled antibody was purified by gel-filtration HPLC on Bio-Sil SEC 400-5 (Bio-Rad Laboratories, Inc., USA) The purified labelled antibody was diluted in (300 mmol/l potassium phosphate, 100 mmol/l NaCl, 10 mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluminescence was measured by using an AutoLumat LB 953 (Berthold Technologies GmbH & Co. KG).

Solid phase antibody (coated antibody):
Solid phase: Polystyrene tubes (Greiner Bio-One International AG, Austria) were coated (18 h at room temperature) with antibody (1.5 µg antibody/0.3 ml 100 mmol/l NaCl, 50 mmol/l Tris/HCl, pH 7.8). After blocking with 5 % bovine serum albumin, the tubes were washed with PBS, pH 7.4 and vacuum dried.

### hGH Immunoassay:

50 µl of sample (or calibrator) was pipetted into coated tubes, after adding labeled antibody (200 ul), the tubes were incubated for 2 h at 18-25 °C. Unbound tracer was removed by washing 5 times (each 1 ml) with washing solution (20 mmol/l PBS, pH 7.4, 0.1 % Triton X-100). Tube-bound labelled antibody was measured by using the LB 953. Using a fixed concentration 1ng/ml of hGH. The signal (RLU at 1ng hGH /ml) to noise (RLU without us-hGH) ratio of different antibody combinations is given in table 1. All antibodies were able to generate a sandwich complex with any other antibody. The antibody pair with strongest signal to noise ratio (best sensitivity) was subsequently used to perform the us-hGH-immunoassay: hGH G12 antibody was used as coated tube antibody and hGH H4 antibody was used as labelled antibody.

**Table 1: Results of noise to ratio determinations between different pairs of hGH antibodies.**

| hGH antibody | Solid phase antibody | H2 | H8 | G12 | H4 | D7 |
|---|---|---|---|---|---|---|
| Labelled antibody | | | | | | |
| H2 | | / | 9.665 | 11.005 | 9.259 | 10.102 |
| H8 | | 8.512 | / | 7.833 | 8.446 | 6.384 |
| G12 | | 10.112 | 9.846 | / | 10.905 | 7.751 |
| H4 | | 11.213 | 8.675 | 12.225 | / | 6.843 |
| D7 | | 2.488 | 2.761 | 3.954 | 2.713 | / |

### Calibration:

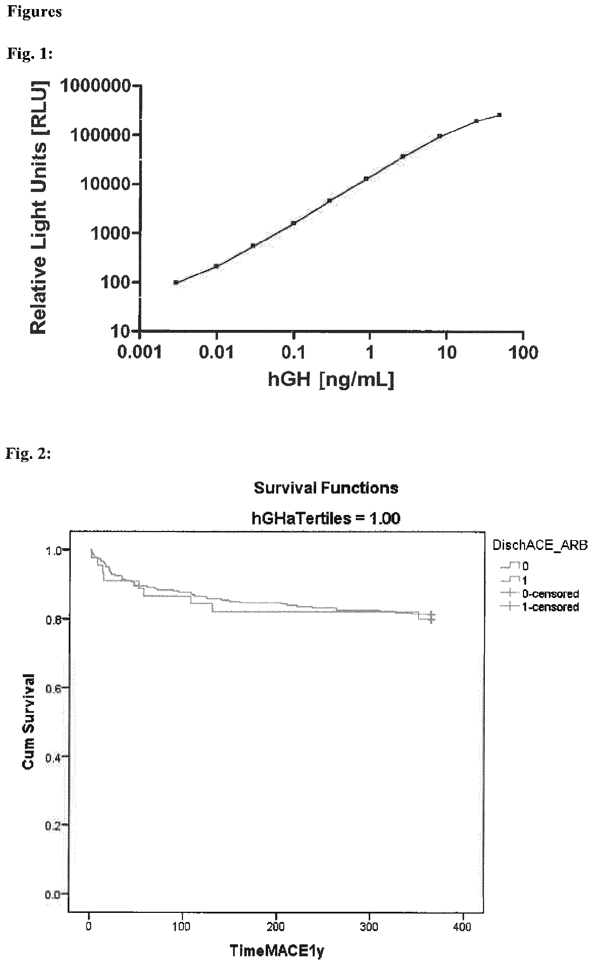

The assay was calibrated, using dilutions of recombinant hGH (WHO International Standard, NIBSC code 98/574), diluted in 20 mM K2PO4, 6 mM EDTA, 0,5% BSA, 50uM Amastatin, 100uM Leupeptin, pH 8.0. (Fig. 1)

### Assay specifications

The analytical assay sensitivity (mean relative light units of 20 determinations of hGH free sample plus 2 S.D.) was 2pg/ml of hGH and the functional assay sensitivity (see above) was 8.5 pg/ml. Recovery and dilution was > 85% intra measurement range of 5-10.000 pg/ml hGH. The coefficient of correlation of N=997 samples between the us-hGH assay and a hGH assay specific for recombinant hGH (22KD) was r=0.98 and a r of 0.95 was found for an assay recognizing preferentially hGH isoforms naturally produced by the pituitary (Bidlingmaier M, Suhr J, Ernst A, Wu Z, Keller A, Strasburger CJ, et al. High-sensitivity chemiluminescence immunoassays for detection of growth hormone doping in sports. Clinical chemistry. 2009;55(3):445-53. Epub 2009/01/27). These data are indicating the suitability of all hGH isoform measurements within the present invention.

### Example 2, Study Population:

We studied 953 STEMI and NSTEMI patients admitted to University Hospitals of Leicester NHS trust between August 2004 and April 2007. This observational cohort study complied with the Declaration of Helsinki and was approved by the local ethics committee; written informed consent was obtained from patients. AMI was diagnosed if a patient had a cardiac troponin I level above the 99th centile with at least one of the following: chest pain lasting >20 minutes or diagnostic serial electrocardiographic changes consisting of new pathological Q waves or ST-segment and T-wave changes. Patients with known malignancy, renal replacement therapy or surgery in the previous month were excluded. Estimated glomerular filtration rate (eGFR) was calculated from the simplified Modification of Diet in Renal Disease formula (Smilde TD, van Veldhuisen DJ, Navis G, Voors AA, Hillege HL. Drawbacks and prognostic value of formulas estimating renal function in patients with chronic heart failure and systolic dysfunction. Circulation 2006;114:1572-80). All patients received standard medical treatment and revascularisation was at the discretion of the attending physician.

Plasma samples: Blood samples (anticoagulated with EDTA and aprotinin) were drawn after 15 minutes bed rest, immediately after diagnosis and within 36 h of symptom onset. Plasma was stored at -80°C until assayed in a single batch for blinded determination of plasma hGH.

### Example 3, Analysis

The efficacy of treatment of patients with acute myocardial infarction with various drugs was analysed depending on the patients' hGH concentration, which was determined within a timeframe of maximally 36 hours after symptom onset. The drugs analysed were a) Angiotensin converting enzyme inhibitor (ACE) or Angiotensin Receptor Blocker (ARB), and b) beta-blocker. The endpoint for determining efficacy was occurrence of major adverse cardiac events (MACE) within one year after symptom onset. MACE was defined as a combination of the following endpoints: all-cause mortality, hospitalization due to heart failure, or re-acute myocardial infarction (re-AMI).

### Angiotensin converting enzyme inhibitor (ACE) or Angiotensin Receptor Blocker (ARB)

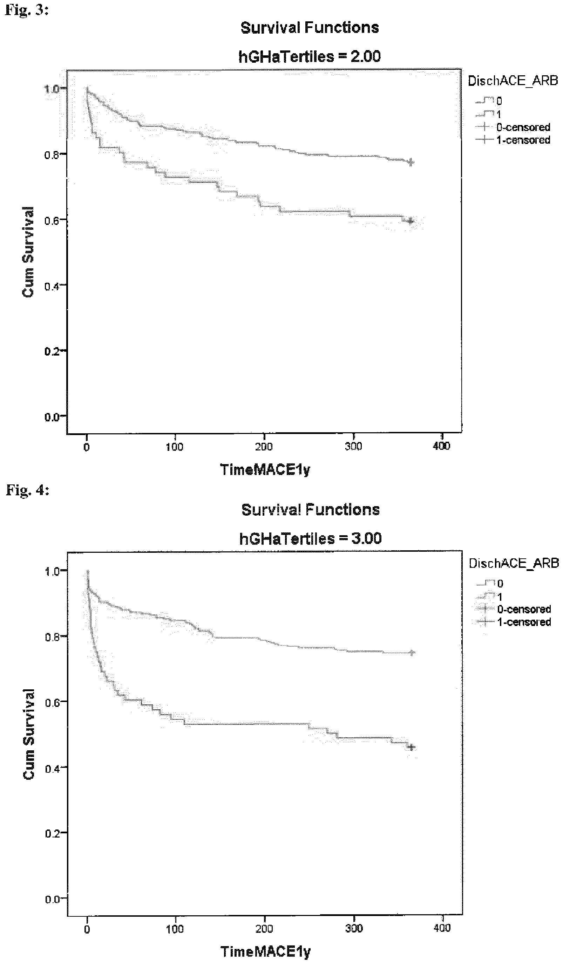

The patient cohort consisted of 953 cases with 245 MACE endpoints within 1 year. Various clinical variables were associated with prediction of MACE within one year, as determined by Cox regression analysis (table 1). When hGH concentrations were added to the statistical model (table 2), hGH was an independent predictor of MACE, and, surprisingly, also the interaction of hGH and treatment with ACE/ARB. Kaplan-Meier analysis for the occurrence of MACE depending on treatment with ACE/ARB was performed for the patient population after separation into hGH tertiles, that is patient with low (<330 pg/mL), medium (330 - 1370 pg/mL) and high concentrations (> 1370 pg/mL) of hGH. The number of MACE for the subgroups are shown in table 3. As shown in table 4, the ACE/ARB treatment effect on MACE was dependent on hGH tertiles and significant only for hGH tertiles 2 and 3, that is for medium and high hGH concentrations. The effect was more pronounced in hGH tertile 3 than in hGH tertile 2. Fig. 2-4 show respective Kaplan-Meier-Plots for the three hGH tertiles.

### Variables in the Equation

**Table 1: Multivariate Cox regression analysis for the prediction of MACE within 1 year.**

| | B | SE | Wald | df | Sig. | Exp(B) |
|---|---|---|---|---|---|---|
| age | .032 | .006 | 29.831 | 1 | .000 | 1.032 |
| sex | .138 | .119 | 1.360 | 1 | .244 | 1.148 |
| PMHmi_angina | .257 | .115 | 5.024 | 1 | .025 | 1.293 |
| pmhbp | .284 | .114 | 6.162 | 1 | .013 | 1.329 |
| PMHdiabetes | .265 | .119 | 4.957 | 1 | .026 | 1.304 |
| KillipAbove1 | .493 | .116 | 17.922 | 1 | .000 | 1.637 |
| eGFR | -.015 | .003 | 20.395 | 1 | .000 | .985 |
| ClassSTEMI | .307 | .115 | 7.077 | 1 | .008 | 1.359 |
| DischACE_ARB | -.369 | .116 | 10.142 | 1 | .001 | .692 |

### Variables in the Equation

**Table 2: Multivariate Cox regression analysis for the prediction of MACE within 1 year including hGH and interaction with ACE/ARB therapy.**

| | B | SE | Wald | df | Sig. | Exp(B) |
|---|---|---|---|---|---|---|
| age | .033 | .007 | 21.337 | 1 | .000 | 1.033 |
| sex | .046 | .144 | .102 | 1 | .749 | 1.047 |
| PMHmi_angina | .038 | .143 | .071 | 1 | .789 | 1.039 |
| pmhbp | .123 | .140 | .768 | 1 | .381 | 1.131 |
| PMHdiabetes | .267 | .145 | 3.375 | 1 | .066 | 1.305 |
| KillipAbove1 | .553 | .138 | 16.070 | 1 | .000 | 1.739 |
| eGFR | -.019 | .004 | 19.937 | 1 | .000 | .981 |
| ClassSTEMI | .636 | .145 | 19.167 | 1 | .000 | 1.889 |
| DischACE_ARB | -.591 | .162 | 13.243 | 1 | .000 | .554 |
| ZLoghGH | .375 | .145 | 6.658 | 1 | .010 | 1.455 |
| DischACE_ARB*ZLoghG H | -.359 | .166 | 4.657 | 1 | .031 | .698 |

### Case Processing Summary

**Table 3: Case Processing Summary for MACE depending on ACE/ARB treatment in patients with low (tertile 1), medium (tertile 2) and high (tertile 3) concentrations of hGH.**

| | | | | Censored | |
|---|---|---|---|---|---|
| hGHaTertiles | DischACE ARB | Total N | N of Events | N | Percent |
| 1.00 | 0 | 45 | 9 | 36 | 80.0% |
| | 1 | 270 | 50 | 220 | 81.5% |
| | Overall | 315 | 59 | 256 | 81.3% |
| 2.00 | 0 | 66 | 27 | 39 | 59.1% |
| | 1 | 257 | 59 | 198 | 77.0% |
| | Overall | 323 | 86 | 237 | 73.4% |
| 3.00 | 0 | 68 | 37 | 31 | 45.6% |
| | 1 | 247 | 63 | 184 | 74.5% |
| | Overall | 315 | 100 | 215 | 68.3% |
| Overall | Overall | 953 | 245 | 708 | 74.3% |

### Pairwise Comparisons

**Table 4: Statistical comparison of ACE/ARB treatment effect on MACE depending on hGH tertiles.**

| | | | 0 | | 1 | |
|---|---|---|---|---|---|---|
| | hGHaTertiles | DischACE ARB | Chi-Square | Sig. | Chi-Square | Sig. |
| Log Rank (Mantel-Cox) | 1.00 | 0 | | | .070 | .791 |
| | | 1 | .070 | .791 | | |
| | 2.00 | 0 | | | 10.492 | .001 |
| | | 1 | 10.492 | .001 | | |
| | 3.00 | 0 | | | 25.970 | .000 |
| | | 1 | 25.970 | .000 | | |

### Beta-blockers

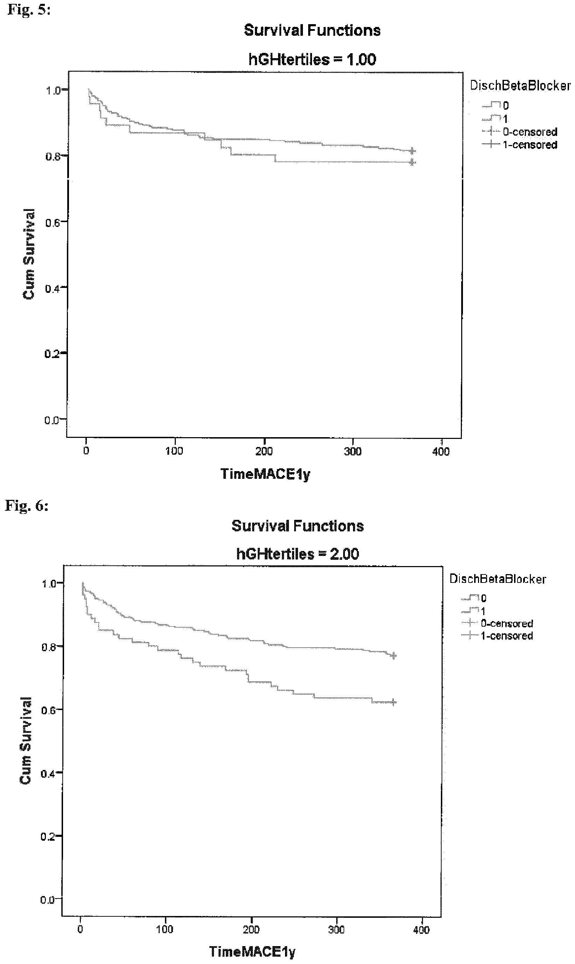

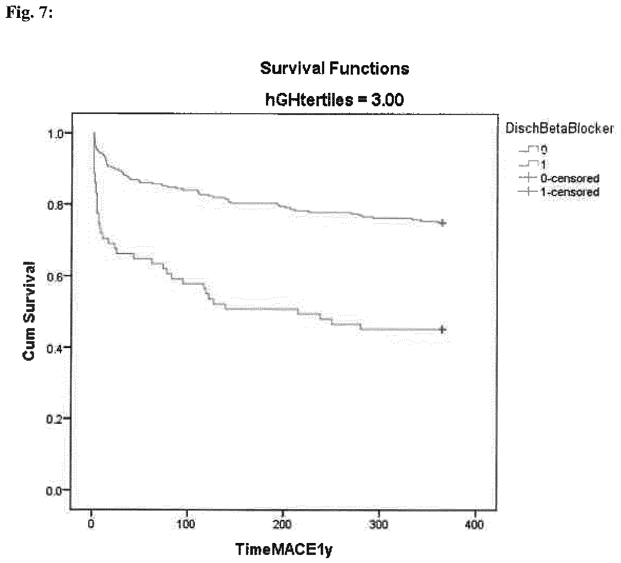

In the same patient cohort, multivariate Cox regression analysis revealed also a significant interaction of hGH and treatment with Beta-blockers for prediction of 1-year MACE (table 5). Kaplan-Meier analysis for the occurrence of MACE depending on treatment with Beta-blockers was performed for the patient population after separation into hGH tertiles, that is patient with low, medium and high concentrations of hGH. The number of MACE for the subgroups are shown in table 6. As shown in table 7, the Beta-blockers treatment effect on MACE was dependent on hGH tertiles and significant only for hGH tertiles 2 and 3, that is for medium and high hGH concentrations. The effect was more pronounced in hGH tertile 3 than in hGH tertile 2. Fig.5-7 show respective Kaplan-Meier-Plots for the three hGH tertiles.

### Variables in the Equation

**Table 5: Multivariate Cox regression analysis for the prediction of MACE within 1 year including hGH and interaction with Beta-blocker therapy.**

| | B | SE | Wald | df | Sig. | Exp(B) |
|---|---|---|---|---|---|---|
| age | .031 | .007 | 17.475 | 1 | .000 | 1.031 |
| sex | .086 | .145 | .356 | 1 | .551 | 1.090 |
| PMHmi_angina | .082 | .141 | .337 | 1 | .562 | 1.085 |
| pmhbp | .076 | .140 | .291 | 1 | .590 | 1.079 |
| PMHdiabetes | .282 | .147 | 3.671 | 1 | .055 | 1.326 |
| KillipAbove1 | .528 | .139 | 14.540 | 1 | .000 | 1.696 |
| eGFR | -.021 | .004 | 23.658 | 1 | .000 | .979 |
| ClassSTEMI | .573 | .144 | 15.936 | 1 | .000 | 1.774 |
| DischBetaBlocker | -.383 | .157 | 5.933 | 1 | .015 | .682 |
| ZLoghGH | .415 | .153 | 7.348 | 1 | .007 | 1.514 |
| DischBetaBlocker*ZLogh GH | -.399 | .172 | 5.365 | 1 | .021 | .671 |

### Case Processing Summary

**Table 6: Case Processing Summary for MACE depending on Beta-blocker treatment in patients with low (tertile 1), medium (tertile 2) and high (tertile 3) concentartions of hGH.**

| | | | | Censored | |
|---|---|---|---|---|---|
| hGHtertiles | DischBetaBlocker | Total N | N of Events | N | Percent |
| 1.00 | 0 | 46 | 10 | 36 | 78.3% |
| | 1 | 269 | 49 | 220 | 81.8% |
| | Overall | 315 | 59 | 256 | 81.3% |
| 2.00 | 0 | 80 | 30 | 50 | 62.5% |
| | 1 | 242 | 55 | 187 | 77.3% |
| | Overall | 322 | 85 | 237 | 73.6% |
| 3.00 | 0 | 71 | 39 | 32 | 45.1% |
| | 1 | 244 | 61 | 183 | 75.0% |
| | Overall | 315 | 100 | 215 | 68.3% |
| Overall | Overall | 952 | 244 | 708 | 74.4% |

### Pairwise Comparisons

**Table 7: Statistical comparison of Beta-blocker treatment effect on MACE depending on hGH tertiles.**

| | | | 0 | | 1 | |
|---|---|---|---|---|---|---|
| | hGHtertiles | DischBetaBlocker | Chi-Square | Sig. | Chi-Square | Sig. |
| Log Rank (Mantel-Cox) | 1.00 | 0 | | | .359 | .549 |
| | | 1 | .359 | .549 | | |
| | 2.00 | 0 | | | 7.355 | .007 |
| | | 1 | 7.355 | .007 | | |
| | 3.00 | 0 | | | 29.334 | .000 |
| | | 1 | 29.334 | .000 | | |

Characteristics of the 953 AMI patients according to hGH tertiles on admission are given in table 8.

**Table 8: Characteristics of the 953 AMI patients according to h-GH tertiles on admission. Numerical data are presented as n (%). P values are quoted for the Kruskal Wallis or Chi squared tests for continuous or categorical variables respectively. Numbers (%) or Mean± SD are reported.**

| | | hGH tertiles (pg/mL) | | | |
|---|---|---|---|---|---|
| | All | 1 <330 | 2 330-1370 | 3 >1370 | P Value |
| | n=953 | n=315 | n=323 | n=315 | |
| hGH (pg/mL) | 1700 ± 2850 | 140 ± 80 | 750 ± 0.310 | 4240 ± 3850 | <0.0005 |
| **Demographics** | | | | | |
| Age (years) | 66.1 ± 12.8 | 61.9 ± 11.9 | 67.3 ± 12.6 | 69.0 ± 12.9 | <0.0005 |
| Male (%) | 687 (72) | 262 (83) | 221 (68) | 204 (65) | <0.0005 |
| ST elevation AMI | 459 (48) | 139 (44) | 154 (48) | 166 (53) | NS |
| Previous History | | | | | |
| IHD | 320 (34) | 103 (33) | 117 (36) | 100 (32) | NS |
| Heart Failure | 37(4) | 9 (3) | 12 (4) | 16 (5) | NS |
| Hypertension | 493 (52) | 149 (47) | 166 (52) | 178 (57) | NS |
| Diabetes Mellitus | 227 (24) | 74 (23) | 76 (24) | 77 (24) | NS |
| Killip Class>1 | 390 (41) | 112 (36) | 138 (43) | 140 (45) | <0.05 |
| Glucose (mmol/L) | 8.8 ± 4.2 | 8.3 ± 3.4 | 8.8 ± 3.8 | 9.5 ± 4.9 | <0.021 |
| Troponin I (µg/L) | 12.5 ± 24.6 | 9.2 ± 20.3 | 14.0 ± 25.6 | 14.4 ± 27.2 | <0.0005 |
| eGFR (ml/min/1.73m²) | 66.2 ± 19.9 | 71.1 ± 16.9 | 64.1 ± 19.0 | 63.5 ± 22.4 | <0.0005 |

| **Treatment** | | | | | |
|---|---|---|---|---|---|
| Aspirin | 794 (83) | 285 (90) | 260(80) | 249 (79) | <0.0005 |
| Beta-blocker | 755 (79) | 269 (85) | 242 (75) | 244 (77) | 0.004 |
| ACE inhibitor or ARB * | 774 (81) | 270 (86) | 257 (80) | 247 (78) | 0.041 |
| Statin | 824 (86) | 287 (91) | 278 (86) | 259(82) | 0.005 |
| Loop Diuretic | 242 (25) | 57 (18) | 92 (29) | 93 (30) | 0.001 |
| Revascularisation | 241 (25) | 95 (30) | 76 (24) | 70 (22) | NS |

| | | | | | |
|---|---|---|---|---|---|
| *ARB = Angiotensin 2 receptor blocker | | | | | |

During follow-up over 2 years, there were 281 MACE, the primary composite endpoint (comprising 117 deaths, 71 HF hospitalisations and 93 re-AMIs). Patients with MACE had higher levels of hGH on presentation (median [range], 910 [40-26 280] pg/mL) compared to event free survivors (590 [20-21 600], p<0.0005 using the Mann -Whitney test). Table 8 also illustrates the higher prevalence of MACE in patients with higher hGH levels.

Table 9 reports the univariate hazard ratios of various factors, therapies and biomarkers that affected the outcome of MACE at 2 years. In multivariate analysis, individual therapies and their interaction with hGH levels were examined. Age, Killip class>1, eGFR were retained in all models as independent predictors, together with hGH levels. Beta blocker therapy was associated with lower MACE (p=0.03) and showed a significant interaction with GH levels (p=0.047, Table 9). Therapy with ACE/ARB also showed a significant interaction with hGH levels (p=0.016, Table 9).

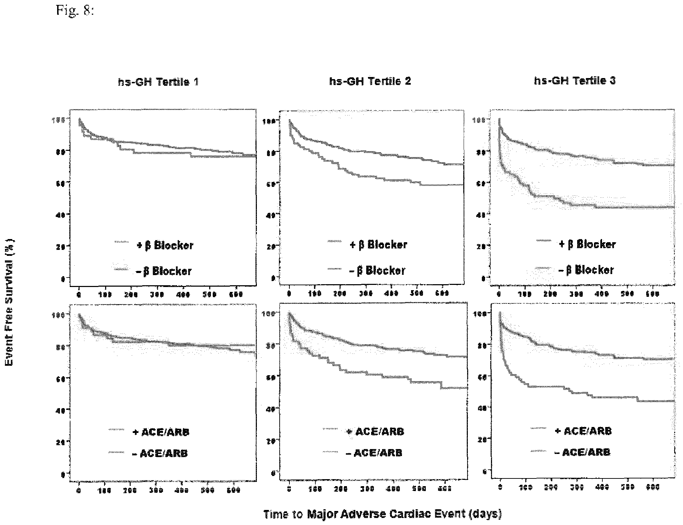

Kaplan-Meier survival analysis was used to visualise the interactions of therapies with beta blocker or ACE/ARB according to hGH tertiles (Figure 8). For beta blocker therapy, there were significant differences in MACE rates between those prescribed compared to those who were not prescribed this treatment in the 2nd (p=0.009) and 3rd hGH tertiles (p<0.0005). For ACE/ARB treatment, there were significant differences in MACE rates between those prescribed compared to those who were not prescribed this treatment in the 2nd (p=0.001) and 3rd hGH tertiles (p<0.0005). In contrast, for both treatments, there was no difference in MACE rates between those prescribed or not prescribed these treatments in those patients in the lowest hGH tertile.

**Table 9.Cox regression analysis for MACE at 2 years post-AMI. Multivariable analysis results are reported for model 1 and 2 which included clinical variables and hGH, with interaction terms hGH with beta blockers (model 1) or ACE/ARB (model 2).**

| | **Univariable HR (95% CI)** | **P** | **Multivariable Model 1 HR (95% CI)** | **P** | **Multivariable Model 2 HR (95% CI)** | **P** |
|---|---|---|---|---|---|---|
| Age (years) | 1.05 (1.04-1.06) | 0.001 | 1.03 (1.01-1.04) | 0.001 | 1.03 (1.02-1.04) | 0.001 |
| Male Sex | 0.59 (0.46-0.75) | 0.001 | 1.13 (0.84-1.51) | NS | 1.10 (0.83-1.47) | NS |
| ST elevation | 0.97 (0.77-1.23) | NS | 1.27 (0.92-1.76) | NS | 1.31 (0.95-1.81) | NS |
| Killip class>1 | 2.62 (2.06-3.33) | 0.001 | 1.66 (1.26-2.19) | 0.001 | 1.67 (1.26-2.20) | 0.001 |
| eGFR (ml min⁻¹ /1.73m²) | 0.97 (0.96-0.97) | 0.001 | 0.99 (0.98-0.99) | 0.006 | 0.98 (0.97-0.99) | 0.001 |
| | | | | | | |

| **Past history** | | | | | | |
|---|---|---|---|---|---|---|
| Ischemic heart disease | 1.67 (1.32-2.11) | 0.001 | 1.06 (0.80-1.42) | NS | 1.03 (0.76-1.38) | NS |
| Hypertension | 1.69 (1.32-2.15) | 0.001 | 1.13 (0.85-1.50) | NS | 1.18 (0.88-1.57) | NS |
| Diabetes | 1.59 (1.23-2.04) | 0.001 | 1.26 (0.94-1.69) | NS | 1.27 (0.95-1.69) | NS |
| | | | | | | |

| **Treatment** | | | | | | |
|---|---|---|---|---|---|---|
| Aspirin | 0.54 (0.41-0.72) | 0.001 | excluded | | excluded | |
| Statin | 0.38 (0.29-0.51) | 0.001 | excluded | | excluded | |
| Loop Diuretic | 2.30 (1.81-2.92) | 0.001 | excluded | | excluded | |
| ACE/ARB | 0.51 (0.39-0.66) | 0.001 | excluded | | 0.74 (0.53-1.04) | NS |
| β blocker | 0.51 (0.39-0.65) | 0.001 | 0.70 (0.52-0.97) | 0.03 | excluded | |
| | | | | | | |

| Biomarkers | | | | | | |
|---|---|---|---|---|---|---|
| Log Troponin (µg/L) | 1.10 (0.97-1.26) | NS | 1.13 (0.93-1.36) | NS | 1.13 (0.93-1.37) | NS |
| Log hGH | 1.76 (1.60-1.94) | 0.001 | 1.43 (1.05-1.95) | 0.026 | 1.49 (1.10-2.02) | 0.01 |
| β blocker * hGH | | | 0.70 (0.49-0.99) | 0.047 | | |
| ACE/ARB * hGH | | | | | 0.65 (0.47-0.93) | 0.016 |

### Example 4, Malmö Diet and Cancer study

The Malmö Diet and Cancer study - cardiovascular cohort (MDC-CC) is a prospective cohort examined in the early 90's. Further details about this study can be found in earlier publications (Berglund et al. 1993. Journal of Internal Medicine 233: 45-51). We performed a cross-sectional analysis of the relationship between hs-GH and intima media thickness (IMT) in MDC-CC. In multiple regression models with IMT as the dependent variable and the standardized value of the natural logarithm of hs-GH as independent we analyzed 4425 individuals from this cohort with values on both hs-GH and the mean IMT in the common carotid artery (IMT_{cca}) and 3397 participants with values on the maximum IMT at the bifurcation (IMT_{bulb}). Separate models were performed for IMT_{cca} and IMT_{bulb} and they were adjusted for either sex and age or a set of traditional cardiovascular risk factors: sex, age, current smoking, systolic blood pressure, anti-hypertensive medication, BMI, LDL-C, HDL-C and diabetes mellitus.

Carotid ultrasound was performed at baseline. In brief, a predefined extent of the right carotid bifurcation was scanned for presence of plaques and the maximum IMT at the bifurcation (IMT_{bulb}) and the mean IMT in the common carotid artery (IMT_{cca}) was measured.In the initial cross-sectional regression model of 3397 (1957 females, 58%) individuals in the MDC-CC hs-GH exhibited a significant positive correlation with IMT_{bulb} in the whole cohort (P=0.003) and in males (P=0.003) (Table 10). This association was essentially unaffected when adjusting for traditional cardiovascular risk factors (P=0.002 and P=0.005 respectively). The results among females were not significant. Fasting levels of hs-GH were not associated with the IMT_{cca} in the 4425 individuals (2658 females, 60%) that were available in this analysis.

Higher fasting levels of hs-GH were associated with an increased IMT in the carotid bulb in males. The relationship between hs-GH and IMT at the carotid bulb in males is in accordance with previous results of higher fasting values of hs-GH being associated to cardiovascular disease, which was also preferentially seen in males (Hallgren et al. 2014. JACC 64: 1452-1460; Maison et al. 1998. BMJ [Clinical Research ed] 316: 1132-1133). IMT_{bulb} is a good measure of atherosclerosis (Naqvi and Lee. 2014. JACC Cardiovasc Imaging 7: 1025-1038), and this finding thus supports a hypothesis of the connection between hs-GH and cardiovascular diseases being mediated by atherosclerosis.

**Table 10: Linear regression with IMT at baseline in the MDC-CC study as dependent variable and hs-GH as independent. One crude model adjusted for sex and age and one model in addition adjusted for traditional cardiovascular risk factors.**

| **Dependent** | **Gender** | **Model** | **Beta** | **95%CI** | **P** |
|---|---|---|---|---|---|
| IMT_{bulb} | ALL | Crude | 0.060 | 0.020 to 0.099 | 0.003 |
| | | Adjusted | 0.066 | 0.025 to 0.106 | 0.002 |
| | MALE | Crude | 0.075 | 0.025 to 0.125 | 0.003 |
| | | Adjusted | 0.074 | 0.022 to 0.125 | 0.005 |
| | FEMALE | Crude | 0.029 | -0.014 to 0.072 | 0.184 |
| | | Adjusted | 0.038 | -0.006 to 0.083 | 0.089 |
| IMT_{cca} | ALL | Crude | -0.012 | -0.046 to 0.021 | 0.477 |
| | | Adjusted | 0.017 | -0.017 to 0.052 | 0.319 |
| | MALE | Crude | 0.011 | -0.033 to 0.054 | 0.634 |
| | | Adjusted | 0.024 | -0.020 to 0.068 | 0.280 |
| | FEMALE | Crude | -0.027 | -0.063 to 0.009 | 0.145 |
| | | Adjusted | 0.001 | -0.036 to 0.038 | 0.967 |

Crude models adjusted for sex and age. Adjusted model adjusted for: sex, age, systolic blood pressure, antihypertensive medication, diabetes mellitus, current smoking, BMI, LDL-C and HDL-C. The β coefficients are expressed as the increment of standardized values of the natural logarithm of IMT per 1 increment of standardized values of the natural logarithm of hs-GH. Abbreviations: IMT_{cca}, mean value of intima media thickness in the common carotid artery. IMT_{bulb}, max value of the intima media thickness at the carotid bifurcation.

### Example 5, BCAPS

### Study description

The β-Blocker Cholesterol-Lowering Asymptomatic Plaque Study (BCAPS) was a randomized, double blind, placebo-controlled, single-center clinical trial that took place between 1994 and 1999. A detailed description of BCAPS can be found in the original paper (Hedblad et al. 2001. Circulation 103: 1721-1726), following here is a brief summary. The original study population consisted of 361 men and 432 women (total n=793) 49 to 70 years of age recruited from MDC-CC. Subjects included in BCAPS had plaque in the right carotid artery but no symptoms of carotid artery disease. Exclusion criteria were: myocardial infarction, angina pectoris or stroke within the previous 3 months; previous surgery in the right carotid artery; regular use of β-blockers or statins; blood pressure >160 (systolic) or >95 (diastolic) mm Hg; total cholesterol > 8.0 mmol/l; hyperglycemia suspected to require insulin treatment; and conditions that in the view of the investigator made the participant unsuitable for the trial.

All participants provided written consent and the study was approved by The Ethics Committee of Lund University.

The study consisted of 4 different treatment groups and the participants were randomized to 1 of the following: placebo/placebo, metoprolol CR/XL (25 mg once daily)/placebo, fluvastatin (40 mg once daily)/placebo or metoprolol CR/XL (25 mg once daily)/fluvastatin (40 mg once daily).

The treatment period was 36-months. Fasting blood samples were drawn at baseline and at 12, 24 and 36 months. HDL-C, total cholesterol and TG were measured according to standard procedures at the Department of Clinical Chemistry, University Hospital of Malmö. LDL-C levels were calculated according to the Friedewald formula. GH levels were measured in stored fasting plasma samples, which were frozen immediately to -80°C at the different examinations. A number of the samples stored in the freezer were missing or had insufficient plasma for the analysis of hs-GH. Subjects in which either the baseline-value of GH or the 12-month value of GH was missing were excluded from further analysis. After this adjustment the study consisted of 472 individuals. All samples were measured using the hs-hGH immunoassay as described above.

### Ultrasound

Carotid ultrasound was performed at baseline, 18 and 36 months (Hedblad et al. 2001. Circulation 103: 1721-1726). In brief, a predefined extent of the right carotid bifurcation was scanned for presence of plaques and the maximum IMT at the bifurcation (IMT_{bulb}) and the mean IMT in the common carotid artery (IMT_{cca}) was measured.

### Statistical analyses

In the analyses where treatment groups are compared, the groups are either the 4 randomization groups or they are divided in to 2 groups which are fluvastatin vs non-fluvastatin (i.e placebo) or metoprolol vs non-metoprolol. Since fasting hs-GH is known to differ between men and women all models were performed separate for men and women as well as combined. All models were adjusted for age, sex (if not already gender separated) and the natural logarithm of the fasting level of hs-GH at baseline.

To investigate if fasting hs-GH was affected by treatment with fluvastatin or metoprolol we performed multiple regression analysis with ΔGH (baseline value subtracted from 12 month value) as the dependent variable and the treatment groups as independent variables.

We then analyzed if hs-GH affects the treatment effect of the different medicines on the IMT in a series of multiple regression models with IMT at 36 months as the dependent variable. In these analyses the larger randomization groups were used to gain more power. An interaction term between the treatment groups multiplied with ΔGH or the natural logarithm of hs-GH, depending on model, was created to evaluate this interaction. A small number of individuals were lost during follow-up and could not complete the ultrasound examination of IMT at 36 months. When analyzing the IMT, the latest IMT-values (i.e. at 18 months) are used for these individuals and the analysis is adjusted for time between IMT-measurements. The models were in addition adjusted for the natural logarithm of IMT at baseline.

All analyses were performed in SPSS (version 22.0.0, SPSS Inc., Chicago, Ill). A 2-sided P-value of less than 0.05 was considered statistically significant.

### Results

Baseline characteristics of the 472 individuals in the BCAPS study population are shown in table 11. The females in all groups had higher values of hs-GH at baseline compared to males. The subjects in which hs-GH could not be measured due to missing samples at either baseline or 12 months did not differ in their baseline characteristics. The missing samples were evenly distributed over the different treatment groups, but generally more men than women were in the group of missing samples. This made the male/female ratio marginally different in two of the four treatment groups (table 11).

In linear regression models the change in levels of hs-GH at 12 months compared to baseline (ΔGH) were related against the different treatment groups (Table 12). When analyzing the whole cohort none of the analyses were significant. In males treatment with fluvastatin/metoprolol had a significant negative correlation with ΔGH compared against placebo (P=0.015). There was also a significant negative correlation between ΔGH and fluvastatin treatment compared to placebo (P=0.046). Among females the correlations were positive and significant for fluvastatin/metoprolol (P=0.010) and borderline significant for fluvastatin (P=0.051).

Male subjects treated with fluvastatin had a greater reduction of hs-GH over 12 months as compared to subjects not treated with fluvastatin. In males, treatment with metoprolol/fluvastatin compared to placebo/placebo was significantly associated with a greater reduction of hs-GH over 12 months of treatment independently of baseline level of hs-GH. Moreover, when all male subjects receiving fluvastatin (metoprolol/fluvastatin and placebo/fluvastatin) were compared with all subjects with no fluvastatin treatment, fluvastatin treatment was also associated with a greater 12 months reduction of hs-GH, suggesting that the fasting value of hs-GH is lowered by treatment with fluvastatin in males. However in this context it should be noted that the change of hs-GH over 12 months in the fluvastatin/placebo-group was not significantly different when compared to the placebo/placebo group.

**Table 11: Clinical characteristics of the study population in BCAPS.**

| **Variable** | **Placebo /Placebo** | **Metoprolol/Placebo** | **Fluvastatin/Metoprolol** | **Fluvastatin/Placebo** |
|---|---|---|---|---|
| Number of participants | 117 | 118 | 117 | 120 |
| Female (%) | 78 (66.7) | 78 (66.1) | 70 (59.8) | 72 (60.0) |
| Age, mean (SD), years | 61.5 (5.7) | 60.3 (5.6) | 62.3 (5.0) | 61.8 (5.4) |
| Height, mean (SD), cm | 168 (10) | 167 (8) | 169 (8) | 169 (9) |
| Baseline | | | | |
| Body Mass Index, Mean (SD), kg/m2 | 25.5 (3.6) | 25.5 (3.6) | 25.1 (2.7) | 25.7 (3.7) |
| LDL-C, mean (SD), mmol/L | 4.07 (0.86) | 4.18 (0.92) | 4.15 (0.88) | 4.16 (0.82) |
| HDL-C, mean (SD), mmol/L | 1.48 (0.40) | 1.39 (0.37) | 1.41 (0.35) | 1.37 (0.35) |
| IMT_{cca}, median (IQR) | 0.88 (0.78-0.96) | 0.88 (0.77-0.99) | 0.86 (0.79-0.98) | 0.86 (0.78-0.97) |
| IMT_{bulb}, median (IQR) | 1.74 (1.46-2.09) | 1.77 (1.49-2.24) | 1.84 (1.52-2.29) | 1.81 (1.55-2.17) |
| Growth Hormone - males, geometric mean, 95%CI, pg/mL | 240 (150-390) | 170 (110-270) | 230 (140-380) | 190 (130-260) |
| Growth Hormone - females, geometric mean, 95%CI, pg/mL | 1260 (970-1660) | 1070 (780-1440) | 1080 (840-1390) | 1210 (940-1570) |

| **12 months** | | | | |
|---|---|---|---|---|
| LDL-C, mean (SD), mmol/L | 4.01 (0.82) | 4.17 (0.87) | 3.21 (0.85) | 3.27 (0.74) |
| Growth Hormone - males, geometric mean, 95%CI, pg/mL | 250 (160-410) | 170 (110-280) | 220 (150-340) | 160 (110-230) |
| Growth Hormone - females, geometric mean, 95%CI, pg/mL | 830 (640-1090) | 860 (660-1130) | 1090 (820-1460) | 810 (610-1070) |

| **36 months** | | | | |
|---|---|---|---|---|
| IMT_{cca}, median (IQR) | 0.91 (0.82-1.04) | 0.90 (0.83-1.02) | 0.87 (0.78-0.98) | 0.87 (0.77-0.99) |
| IMT_{bulb}, median (IQR) | 1.98 (1.71-2.35) | 1.89 (1.60-2.43) | 1.99 (1.60-2.44) | 2.02 (1.68-2.30) |

| | | | | |
|---|---|---|---|---|
| Abbreviations: LDL-C, Low-density lipoprotein cholesterol; HDL-C, High-density lipoprotein cholesterol; IMT, intima media thickness. Missing values in IMTbulb: placebo/placebo, n=3; metoprolol/placebo n=3; Fluvastatin/Metoprolol, n=5; fluvastatin/placebo, n=8. Missing values in HDL-C and LDL-C: placebo/placebo, n=1; metoprolol/fluvastatin n=1. | | | | |

**Table 12: Multiple linear regression models of ΔGH (12 months - baseline) vs different treatment groups.**

| **Gender** | **Model** | **Treatment group** | **B** | **95%CI** | **P** |
|---|---|---|---|---|---|
| ALL | All groups | Metoprolol- Placebo | -0.11 | -0.53 to 0.31 | 0.600 |
| | | Fluvastatin-Metoprolol | 0.25 | -0.17 to 0.67 | 0.241 |
| | | Fluvastatin-Placebo | -0.11 | -0.53 to 0.31 | 0.595 |
| | All Fluvastatin | Fluvastatin | 0.12 | -0.18 to 0.42 | 0.423 |
| | All Metoprolol | Metoprolol | 0.13 | -0.17 to 0.43 | 0.399 |
| MALE | All groups | Metoprolol- Placebo | -0.26 | -0.79 to 0.28 | 0.342 |
| | | Fluvastatin-Metoprolol | -0.64 | -1.16 to -0.13 | 0.015 |
| | | Fluvastatin-Placebo | -0.36 | -0.88 to 0.15 | 0.165 |
| | All Fluvastatin | Fluvastatin | -0.37 | -0.73 to -0.01 | 0.046 |
| | All Metoprolol | Metoprolol | -0.27 | -0.63 to 0.10 | 0.150 |
| FEMALE | All groups | Metoprolol- Placebo | -0.06 | -0.63 to 0.51 | 0.842 |
| | | Fluvastatin-Metoprolol | 0.77 | 0.185 to 1.35 | 0.010 |
| | | Fluvastatin-Placebo | 0.02 | -0.56 to 0.59 | 0.956 |
| | All Fluvastatin | Fluvastatin | 0.41 | 0.00 to 0.83 | 0.051 |
| | All Metoprolol | Metoprolol | 0.33 | -0.09 to 0.74 | 0.119 |

Models adjusted for: age and standardized values of natural logarithm of hs-GH at baseline. In addition adjusted for sex in the subgroup all.

Three different models are executed: in "all groups" the different treatment groups are each one compared against placebo. In "all fluvastatin" the individuals receiving fluvastatin are compared against individuals not receiving fluvastatin and vice versa in "all metoprolol". B coefficients are expressed as the increment of ΔGH with treatment of the medicine in question as compared to placebo.

### Figure description

Fig. 1: shows a typical us-hGH assay dose/ signal curve.
Fig. 2: Kaplan-Meier Plot for efficacy of ACE/ARB treatment on MACE in patients with hGH concentrations in tertile 1.
Fig. 3: Kaplan-Meier Plot for efficacy of ACE/ARB treatment on MACE in patients with hGH concentrations in tertile 2.
Fig. 4: Kaplan-Meier Plot for efficacy of ACE/ARB treatment on MACE in patients with hGH concentrations in tertile 3.
Fig. 5: Kaplan-Meier Plot for efficacy of Beta-blocker treatment on MACE in patients with hGH concentrations in tertile 1.
Fig. 6: Kaplan-Meier Plot for efficacy of Beta-blocker treatment on MACE in patients with hGH concentrations in tertile 2.
Fig. 7: Kaplan-Meier Plot for efficacy of Beta-blocker treatment on MACE in patients with hGH concentrations in tertile 3.
Fig. 8: Kaplan-Meier Plot for efficacy of Beta-blocker or ACE/ARB treatment on MACE in patients with hGH concentrations in tertile 1, 2 and 3, respectively.

## Claims

1. Method of determining whether a subject has a vascular risk that can be reduced by blood pressure lowering therapy with antihypertensive drugs comprising the steps:
• determining the level of hGH and/or its isoforms selected from the group comprising hGH isoform 1, hGH isoform 2, hGH isoform 3 and hGH isoform 4 in a blood sample of said subject and
• comparing the determined level of hGH and/or said isoforms thereof in said blood sample with a pre-determined threshold and
• wherein in case the determined level of hGH and/or said isoforms thereof is above said pre-determined threshold then the subject is identified as having a vascular risk that can be reduced by blood pressure lowering therapy with antihypertensive drugs and
• wherein in case the determined level of hGH and/or said isoforms thereof is below said pre-determined threshold then the subject is identified as not having a vascular risk that can be reduced by blood pressure lowering therapy with antihypertensive drugs.

2. Method of determining whether a subject has a vascular risk that can be reduced by blood pressure lowering therapy with antihypertensive drugs according to claim 1, wherein said subject does not have hypertension, and wherein having hypertension is defined as having blood pressure of at least > 140 mmHG (systolic) to 90 mmHG (diastolic).

3. Method of determining whether a subject has a vascular risk that can be reduced by blood pressure lowering therapy with antihypertensive drugs according to claim 1 or 2, wherein the blood sample is taken from a subject that has not yet been treated with antihypertensive drugs.

4. Method of determining whether a subject has a vascular risk that can be reduced by blood pressure lowering therapy with antihypertensive drugs according to claim 1, 2 or 3, wherein reducing the vascular risk by lowering blood pressure in a subject means preventing a major cardiovascular event and/or a cardiovascular disease in said subj ect.

5. Method of determining whether a subject has a vascular risk that can be reduced by blood pressure lowering therapy with antihypertensive drugs according to any of claims 1 to 4, wherein said subject has never had a major cardiovascular event and has never had any cardiovascular disease.

6. Method of determining whether a subject has a vascular risk that can be reduced by blood pressure lowering therapy with antihypertensive drugs according to any of the claims 1 to 4, wherein said subject has suffered an acute myocardial infarction or acute heart failure within the last 2 weeks, preferably within the last one week, preferably within the last 36 hours.

7. Method of determining whether a subject has a vascular risk that can be reduced by blood pressure lowering therapy with antihypertensive drugs according to any of the claims 1 to 6, wherein said antihypertensive drug is selected from the group comprising Angiotensin converting enzyme inhibitor (ACE inhibitor), Angiotensin Receptor Blocker (ARB), and Beta-Adrenoreceptor Blocker (Beta-blocker), statin or combinations thereof.

8. Method of determining whether a subject has a vascular risk that can be reduced by blood pressure lowering therapy with antihypertensive drugs according to any of the preceding claims, wherein the blood sample is selected from the group comprising whole blood, serum, and plasma.

9. Method of determining whether a subject has a vascular risk that can be reduced by blood pressure lowering therapy with antihypertensive drugs according to any of the preceding claims, wherein the blood sample is a fasting sample.

10. Method of determining whether a subject has a vascular risk that can be reduced by blood pressure lowering therapy with antihypertensive drugs according to any of the preceding claims, wherein the measurement method that is used to determine the level of hGH and/or its isoforms selected from the group comprising hGH isoform 1, hGH isoform 2, hGH isoform 3 and hGH isoform 4 has a sensitivity of at least 50 pg/ml.

11. Method of determining whether a subject has a vascular risk that can be reduced by blood pressure lowering therapy with antihypertensive drugs according to any of the preceding claims, wherein the measurement method that is used to determine the level of hGH and/or its isoforms selected from the group comprising hGH isoform 1, hGH isoform 2, hGH isoform 3 and hGH isoform 4 is an immunoassay having an assay sensitivity of at least 50 pg/ml.

12. Method of determining whether a subject has a vascular risk that can be reduced by blood pressure lowering therapy with antihypertensive drugs according to any of the preceding claims, wherein the pre-determined threshold is 330 pg/ml or above.

13. Method of determining whether a subject has a vascular risk that can be reduced by blood pressure lowering therapy with antihypertensive drugs according any of the claims 1-12, wherein the level of hGH and/or its isoforms selected from the group comprising hGH isoform 1, hGH isoform 2, hGH isoform 3 and hGH isoform 4 is determined by an immunoassay that comprises at least two antibodies that bind to hGH and/or its isoforms selected from the group comprising hGH isoform 1, hGH isoform 2, hGH isoform 3 and hGH isoform 4.

14. Method of determining whether a subject has a vascular risk that can be reduced by blood pressure lowering therapy with antihypertensive drugs according to claim 13, wherein each of the at least two antibodies that bind to hGH and/or its isoforms selected from the group comprising hGH isoform 1, hGH isoform 2, hGH isoform 3 and hGH isoform 4 have an affinity towards hGH and/or said isoforms thereof that is at least 10⁸M⁻¹.

15. Method of determining whether a subject has a vascular risk that can be reduced by blood pressure lowering therapy with antihypertensive drugs according to any of the preceding claims, wherein said method is used for prevention of a major cardiovascular event and/or a cardiovascular disease in said subject selected from the group comprising heart failure, atherosclerosis, hypertension, cardiomyopathy, myocardial infarction and stroke.

16. Method of determining whether a subject has a vascular risk that can be reduced by blood pressure lowering therapy with antihypertensive drugs according to any of the preceding claims, wherein the method further comprises:
• recommendation of administration of antihypertensive drugs to a subject whose determined level of hGH and/or its isoforms is above said pre-determined threshold and who is identified as having a vascular risk that can be reduced by blood pressure lowering therapy with antihypertensive drugs or
• recommendation of withholding with antihypertensive drugs to a subject whose determined level of hGH and/or its isoforms is below said pre-determined threshold and who is identified as not having a vascular risk that can be reduced by blood pressure lowering therapy with antihypertensive drugs.

17. An antihypertensive drug for use in a method of preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of any of claims 1-16 as having a vascular risk that can be reduced by blood pressure lowering therapy with antihypertensive drugs, wherein said cardiovascular event is selected from the group comprising myocardial infarction, acute heart failure, stroke and cardiovascular death.

18. An antihypertensive drug for use in a method of preventing a major cardiovascular event and/or a cardiovascular disease in a subject whose determined level of hGH and/or its isoforms selected from the group comprising hGH isoform 1, hGH isoform 2, hGH isoform 3 and hGH isoform 4 is above said pre-determined threshold and wherein said subject is identified according to the methods of any of claims 1-16 as having a vascular risk that can be reduced by blood pressure lowering therapy with antihypertensive drugs, wherein said subject does not have hypertension.

19. A method for monitoring the therapy response of a subject at vascular risk that is treated with antihypertensive drugs comprising the steps:
• determining the level of hGH and/or its isoforms selected from the group comprising hGH isoform 1, hGH isoform 2, hGH isoform 3 and hGH isoform 4 in a blood sample of said subject taken at a time point XI with antihypertensive drugs and
• determining the level of hGH and/or said isoforms thereof in a blood sample of said subject taken at a time point X2 with antihypertensive drugs, and
• wherein time point XI is either before the beginning of therapy or after starting the therapy but in any event earlier than time point X2, and
• wherein time point X2 is after starting the therapy and in any event later than time point XI
• comparing the determined level of hGH and/or said isoforms thereof in said blood sample taken at point XI with the determined level of hGH and/or said isoforms thereof in said blood sample taken at time point X2
• wherein a lower level of hGH and/or said isoforms thereof in said blood sample taken at time point X2 compared to the level of hGH and/or said isoforms thereof in said blood sample taken at time point XI indicates a therapy response.

20. A method for monitoring the therapy response of a subject at vascular risk that is treated with antihypertensive drugs according to claim 19, wherein the determined level of hGH and/or its isoforms selected from the group comprising hGH isoform 1, hGH isoform 2, hGH isoform 3 and hGH isoform 4 in said blood sample taken at point XI is above a pre-determined threshold and the subject has been identified as having a vascular risk that can be reduced by blood pressure lowering therapy with antihypertensive drugs.

21. A method for monitoring the therapy response of a subject at vascular risk that is treated with antihypertensive drugs according to claim 19 or 20, wherein the time point XI is before starting the therapy.

22. A method for monitoring the therapy response of a subject at vascular risk that is treated with antihypertensive drugs according to any of claims 19 to 21, wherein time point X2 is 12 months, 24 months, or 36 months after time point X1.

## Patentansprüche

1. Verfahren zur Bestimmung, ob bei einem Subjekt ein vaskuläres Risiko besteht, welches durch eine blutdrucksenkende Therapie mit blutdrucksenkenden Medikamenten reduziert werden kann, wobei das Verfahren die folgenden Schritte umfasst:
• Bestimmen des Werts von hGH und/oder dessen Isoformen, ausgewählt aus der Gruppe bestehend aus hGH-Isoform 1, hGH-Isoform 2, hGH-Isoform 3 und hGH-Isoform 4, in einer Blutprobe des Subjekts und
• Vergleichen des bestimmten Wertes von hGH und/oder dessen Isoformen in der Blutprobe mit einem vorbestimmten Schwellenwert und
• wobei, wenn der bestimmte Wert von hGH und/oder den Isoformen davon über dem vorbestimmten Schwellenwert liegt, bei dem Subjekt ein vaskuläres Risiko identifiziert ist, welches durch eine blutdrucksenkende Therapie mit blutdrucksenkenden Medikamenten reduziert werden kann,
und
• wobei, wenn der bestimmte Wert von hGH und/oder den Isoformen davon unter dem vorbestimmten Schwellenwert liegt, bei dem Subjekt kein vaskuläres Risiko identifiziert ist, welches durch eine blutdrucksenkende Therapie mit blutdrucksenkenden Medikamenten reduziertwerden kann.

2. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach Anspruch 1, wobei bei dem Subjekt keine Hypertonie vorliegt und wobei das Vorliegen einer Hypertonie definiert ist als das Vorliegen eines Blutdrucks von mindestens > 140 mmHG (systolisch) bis 90 mmHG (diastolisch).

3. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach Anspruch 1 oder 2, wobei die Blutprobe von einem Subjekt entnommen wird, das noch nicht mit blutdrucksenkenden Medikamenten behandelt worden ist.

4. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach Anspruch 1, 2 oder 3, wobei eine Reduzierung des vaskulären Risikos durch Senken des Blutdrucks eines Subjekts die Vermeidung eines schwerwiegenden kardiovaskulären Ereignisses und/oder einer kardiovaskulären Erkrankung bei dem Subjekt bedeutet.

5. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der Ansprüche 1 bis 4, wobei das Subjekt noch nie ein schwerwiegendes kardiovaskuläres Ereignis erlitten hat und noch nie eine kardiovaskuläre Erkrankung hatte.

6. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der Ansprüche 1 bis 4, wobei das Subjekt innerhalb der vergangenen 2 Wochen, bevorzugt innerhalb der vergangenen 1 Woche, bevorzugt in den vergangenen 36 Stunden einen akuten Myokardinfarkt oder ein akutes Herzversagen erlitten hat.

7. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der Ansprüche 1 bis 6, wobei das blutdrucksenkende Medikament ausgewählt ist aus der Gruppe bestehend aus Angiotensin-Converting-Enzym-Hemmer (ACE-Hemmer), Angiotensin-Rezeptor-Blocker (ARB) und Beta-Adrenorezeptor-Blocker (Betablocker), Statin oder Kombinationen davon.

8. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei die Blutprobe ausgewählt ist aus der Gruppe bestehend aus Vollblut, Serum und Plasma.

9. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei die Blutprobe eine Nüchternprobe ist.

10. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei das Messverfahren, welches zur Bestimmung des Wertes von hGH und/oder dessen Isoformen, ausgewählt aus der Gruppe bestehend aus hGH-Isoform 1, hGH-Isoform 2, hGH-Isoform 3 und hGH-Isoform 4, verwendet wird, eine Sensitivität von mindestens 50 pg/ml aufweist.

11. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei das Messverfahren, welches zur Bestimmung des Wertes von hGH und/oder dessen Isoformen, ausgewählt aus der Gruppe bestehend aus hGH-Isoform 1, hGH-Isoform 2, hGH-Isoform 3 und hGH-Isoform 4, verwendet wird, ein Immunassay mit einer Assay-Sensitivität von mindestens 50 pg/ml ist.

12. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei der vorbestimmte Schwellenwert 330 pg/ml oder höher ist.

13. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der Ansprüche 1 bis 12, wobei der Wert von hGH und/oder dessen Isoformen, ausgewählt aus der Gruppe bestehend aus hGH-Isoform 1, hGH-Isoform 2, hGH-Isoform 3 und hGH-Isoform 4, mithilfe eines Immunassays bestimmt wird, welcher mindestens zwei Antikörper umfasst, die an hGH und/oder dessen Isoformen, ausgewählt aus der Gruppe bestehend aus hGH-Isoform 1, hGH-Isoform 2, hGH-Isoform 3 und hGH-Isoform 4, binden.

14. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach Anspruch 13, wobei jeder der mindestens zwei Antikörper, welche an hGH und/oder dessen Isoformen, ausgewählt aus der Gruppe bestehend aus hGH-Isoform 1, hGH-Isoform 2, hGH-Isoform 3 und hGH-Isoform 4, binden, eine Affinität von mindestens 10⁸M⁻¹ zu hGH und/oder dessen Isoformen aufweisen.

15. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei das Verfahren verwendet wird, um bei dem Subjekt ein schwerwiegendes kardiovaskuläres Ereignis und/oder eine kardiovaskuläre Erkrankung, ausgewählt aus der Gruppe bestehend aus Herzversagen, Atherosklerose, Hypertonie, Kardiomyopathie, Myokardinfarkt und Schlaganfall, zu vermeiden.

16. Verfahren zur Bestimmung, ob bei einem Subjekt ein vaskuläres Risiko besteht, welches durch eine blutdrucksenkende Therapie mit blutdrucksenkenden Medikamenten reduziert werden kann, nach einem der vorhergehenden Ansprüche, wobei das Verfahren weiterhin Folgendes umfasst:
• Empfehlung der Verabreichung von blutdrucksenkenden Medikamenten an ein Subjekt, dessen bestimmter Wert von HGH und/oder dessen Isoformen über dem vorbestimmten Schwellenwert liegt, und bei dem ein vaskuläres Risiko identifiziert ist, welches durch eine blutdrucksenkende Therapie mit blutdrucksenkenden Medikamenten reduziertwerden kann, oder
• Empfehlung der Unterlassung der Verabreichung von blutdrucksenkenden Medikamenten bei einem Subjekt, dessen bestimmter Wert von hGH und/oder dessen Isoformen unter dem vorbestimmten Schwellenwert liegt, und bei dem kein vaskuläres Risiko identifiziert ist, welches durch eine blutdrucksenkende Therapie mit blutdrucksenkenden Medikamenten reduziertwerden kann.

17. Blutdrucksenkendes Medikament zur Verwendung in einem Verfahren zur Vermeidung eines schwerwiegenden kardiovaskulären Ereignisses und/oder einer kardiovaskulären Erkrankung bei einem Subjekt, dessen bestimmter Wert von hGH und/oder dessen Isoformen, ausgewählt aus der Gruppe bestehend aus hGH-Isoform 1, hGH-Isoform 2, hGH-Isoform 3 und hGH-Isoform 4, über dem vorbestimmten Schwellenwert liegt, und wobei bei dem Subjekt nach den Verfahren eines der Ansprüche 1 bis 16 ein vaskuläres Risiko identifiziert ist, welches durch eine blutdrucksenkende Therapie mit blutdrucksenkenden Medikamenten reduziertt werden kann, wobei das kardiovaskuläre Ereignis ausgewählt ist aus der Gruppe bestehend aus Myokardinfarkt, akutem Herzversagen, Schlaganfall und kardiovaskulärem Tod.

18. Blutdrucksenkendes Medikament zur Verwendung in einem Verfahren zur Vermeidung eines schwerwiegenden kardiovaskulären Ereignisses und/oder einer kardiovaskulären Erkrankung bei einem Subjekt, dessen bestimmter Wert von hGH und/oder dessen Isoformen, ausgewählt aus der Gruppe bestehend aus hGH-Isoform 1, hGH-Isoform 2, hGH-Isoform 3 und hGH-Isoform 4, über dem vorbestimmten Schwellenwert liegt, und wobei bei dem Subjekt nach den Verfahren eines der Ansprüche 1 bis 16 ein vaskuläres Risiko identifiziert ist, welches durch eine blutdrucksenkende Therapie mit blutdrucksenkenden Medikamenten reduziertwerden kann, wobei bei dem Subjekt keine Hypertonie vorliegt.

19. Verfahren zur Überwachung des Therapieansprechens bei einem Subjekt mit vaskulärem Risiko, welches mit blutdrucksenkenden Medikamenten behandelt wird, wobei das Verfahren folgende Schritte umfasst:
• Bestimmen des Werts von hGH und/oder dessen Isoformen, ausgewählt aus der Gruppe bestehend aus hGH-Isoform 1, hGH-Isoform 2, hGH-Isoform 3 und hGH-Isoform 4, in einer Blutprobe des Subjekts mit blutdrucksenkenden Medikamenten, welche zum Zeitpunkt X1 entnommen wurde, und
• Bestimmen des Werts von hGH und/oder dessen Isoformen in einer Blutprobe des Subjekts mit blutdrucksenkenden Medikamenten, welche zum Zeitpunkt X2 entnommen wurde, und
• wobei der Zeitpunkt X1 entweder vorTherapiebeginn oder nach Therapiebeginn, jedoch in jedem Fall vor dem Zeitpunkt X2 liegt, und
• wobei der Zeitpunkt X2 nach Therapiebeginn und in jedem Fall nach dem Zeitpunkt X1 liegt
• Vergleichen des bestimmten Wertes von hGH und/oder dessen Isoformen in der zum Zeitpunkt X1 entnommenen Blutprobe mit dem bestimmten Wert von hGH und/oder dessen Isoformen in der zum Zeitpunkt X2 entnommenen Blutprobe
• wobei ein geringerer Wert von hGH und/oder dessen Isoformen in der zum Zeitpunkt X2 entnommenen Blutprobe verglichen mit dem Wert von hGH und/oder dessen Isoformen in der zum Zeitpunkt X1 entnommenen Blutprobe ein Therapieansprechen anzeigt.

20. Verfahren zur Überwachung des Therapieansprechens bei einem Subjekt mit vaskulärem Risiko, welches mit blutdrucksenkenden Medikamenten behandelt wird, nach Anspruch 19, wobei der bestimmte Wert von hGH und/oder dessen Isoformen, ausgewählt aus der Gruppe bestehend aus hGH-Isoform 1, hGH-Isoform 2, hGH-Isoform 3 und hGH-Isoform 4, in der zum Zeitpunkt X1 entnommenen Blutprobe über dem vorbestimmten Schwellenwert liegt und bei dem Subjekt ein vaskuläres Risiko identifiziert wurde, welches durch eine blutdrucksenkende Therapie mit blutdrucksenkenden Medikamenten reduziertwerden kann.

21. Verfahren zur Überwachung des Therapieansprechens bei einem Subjekt mit vaskulärem Risiko, welches mit blutdrucksenkenden Medikamenten behandelt wird, nach Anspruch 19 oder 20, wobei der Zeitpunkt X1 vor Therapiebeginn liegt.

22. Verfahren zur Überwachung des Therapieansprechens bei einem Subjekt mit vaskulärem Risiko, welches mit blutdrucksenkenden Medikamenten behandelt wird, nach einem der Ansprüche 19 bis 21, wobei der Zeitpunkt X2 12 Monate, 24 Monate oder 36 Monate nach dem Zeitpunkt X1 liegt.

## Revendications

1. Méthode permettant de déterminer si un sujet présente un risque vasculaire pouvant être réduit par une thérapie d'abaissement de la pression artérielle à l'aide de médicaments antihypertenseurs, ladite méthode comprenant les étapes suivantes :
• détermination du taux de hGH et/ou de ses isoformes choisies parmi le groupe comprenant l'isoforme 1 de hGH, l'isoforme 2 de hGH, l'isoforme 3 de hGH et l'isoforme 4 de hGH dans un échantillon de sang dudit sujet et
• comparaison du taux déterminé de hGH et/ou desdites isoformes de celle-ci dans ledit échantillon de sang à un seuil prédéterminé et
• ce pendant que, dans le cas où le taux déterminé de hGH et/ou desdites isoformes de celle-ci est supérieur audit seuil pré-déterminé, que le sujet présente un risque vasculaire pouvant être réduit par thérapie d'abaissement de la pression artérielle à l'aide de médicaments antihypertenseurs et
• ce pendant que, dans le cas où le taux déterminé de hGH et/ou desdites isoformes de celle-ci est inférieur audit seuil pré-déterminé, que le sujet ne présente pas un risque vasculaire pouvant être réduit par thérapie d'abaissement de la pression artérielle à l'aide de médicaments antihypertenseurs.

2. Méthode permettant de déterminer si un sujet présente un risque vasculaire pouvant être réduit par thérapie d'abaissement de la pression artérielle à l'aide de médicaments antihypertenseurs selon la revendication 1, ledit sujet ne souffrant pas d'hypertension, et l'hypertension étant définie comme une pression artérielle d'au moins >140 mmHG (systolique) à 90 mmHG (diastolique).

3. Méthode permettant de déterminer si un sujet présente un risque vasculaire pouvant être réduit par thérapie d'abaissement de la pression artérielle à l'aide de médicaments antihypertenseurs selon la revendication 1 ou 2, l'échantillon de sang étant prélevé sur un sujet n'ayant pas encore été traité à l'aide de médicaments antihypertenseurs.

4. Méthode permettant de déterminer si un sujet présente un risque vasculaire pouvant être réduit par thérapie d'abaissement de la pression artérielle à l'aide de médicaments antihypertenseurs selon la revendication 1, 2 ou 3, la réduction du risque vasculaire par l'abaissement de la pression artérielle chez un sujet qui signifie la prévention d'un événement cardiovasculaire majeur et/ou d'une maladie cardiovasculaire chez ledit sujet.

5. Méthode permettant de déterminer si un sujet présente un risque vasculaire pouvant être réduit par thérapie d'abaissement de la pression artérielle à l'aide de médicaments antihypertenseurs selon l'une quelconque des revendications 1 à 4, ledit sujet n'ayant jamais subi un événement cardiovasculaire majeur et n'ayant jamais souffert d'une maladie cardiovasculaire.

6. Méthode permettant de déterminer si un sujet présente un risque vasculaire pouvant être réduit par thérapie d'abaissement de la pression artérielle à l'aide de médicaments antihypertenseurs selon l'une quelconque des revendications 1 à 4, ledit sujet ayant subi un infarctus aigu du myocarde ou une insuffisance cardiaque aiguë au cours des 2 dernières semaines, de préférence au cours de la dernière semaine, de préférence au cours des 36 dernières heures.

7. Méthode permettant de déterminer si un sujet présente un risque vasculaire pouvant être réduit par thérapie d'abaissement de la pression artérielle à l'aide de médicaments antihypertenseurs selon l'une quelconque des revendications 1 à 6, ledit médicament antihypertenseur étant choisi parmi le groupe comprenant l'inhibiteur de l'enzyme de conversion de l'angiotensine (inhibiteur ECA), le bloqueur des récepteurs de l'angiotensine (BRA) et le bloqueur des récepteurs bêta-adrénorécepteurs (bêta-bloqueur), la statine ou les combinaisons de ceux-ci.

8. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, l'échantillon de sang étant choisi parmi le groupe comprenant le sang entier, le sérum et le plasma.

9. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, l'échantillon de sang choisi étant un échantillon prélevé à jeun.

10. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, la méthode de mesure utilisée pour déterminer le taux de hGH et/ou de ses isoformes choisies parmi le groupe qui comprend l'isoforme 1 de hGH, l'isoforme 2 de hGH, l'isoforme 3 de hGH, et l'isoforme 4 de hGH présentant une sensibilité d'au moins 50 pg/ml.

11. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, la méthode de mesure utilisée pour déterminer le taux de hGH et/ou de ses isoformes choisies parmi le groupe qui comprend l'isoforme 1 de hGH, l'isoforme 2 de hGH, l'isoforme 3 de hGH, et l'isoforme 4 de hGH étant un immunoessai présentant une sensibilité d'essai d'au moins 50 pg/ml.

12. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, le seuil prédéterminé étant 330 pg/ml ou plus.

13. Méthode permettant de déterminer si un sujet présente un risque vasculaire pouvant être réduit par thérapie d'abaissement de la pression sanguine à l'aide de médicaments antihypertenseurs selon l'une quelconque des revendications 1-12, le taux de hGH et/ou de ses isoformes choisies parmi le groupe qui comprend l'isoforme 1 de hGH, l'isoforme 2 de hGH, l'isoforme 3 de hGH et l'isoforme 4 de hGH étant déterminés par un immunoessai comprenant au moins deux anticorps qui se lient à hGH et/ou à ses isoformes choisies parmi le groupe comprenant l'isoforme 1 de hGH, l'isoforme 2 de hGH, l'isoforme 3 de hGH et l'isoforme 4 de hGH.

14. Méthode permettant de déterminer si un sujet présente un risque vasculaire pouvant être réduit par thérapie d'abaissement de la pression artérielle à l'aide de médicaments antihypertenseurs selon la revendication 13, chacune des au moins deux anticorps qui se lient au hGH et/ou à ses isoformes choisies parmi le groupe qui comprend l'isoforme 1 de hGH, l'isoforme 2 de hGH, l'isoforme 3 de hGH, et l'isoforme 4 de hGH présentant une affinité envers hGH et/ou desdites isoformes de celle-ci qui est au moins 10⁸M⁻¹.

15. Méthode pour déterminer si un sujet présente un risque vasculaire pouvant être réduit par thérapie d'abaissement de la pression sanguine à l'aide de médicaments antihypertenseurs selon l'une quelconque des revendications précédentes, ladite méthode étant utilisée pour la prévention d'un événement cardiovasculaire majeur et/ou d'une maladie cardiovasculaire chez ledit sujet choisi parmi le groupe comprenant l'insuffisance cardiaque, l'athérosclérose, l'hypertension, la cardiomyopathie, l'infarctus du myocarde et l'accident vasculaire cérébral.

16. Méthode permettant de déterminer si un sujet présente un risque vasculaire pouvant être réduit par thérapie d'abaissement de la pression artérielle à l'aide de médicaments antihypertenseurs selon l'une quelconque des revendications précédentes, la méthode comprenant en outre :
• la recommandation de l'administration de médicaments antihypertenseurs à un sujet dont le taux déterminé de hGH et/ou de ses isoformes est supérieur audit seuil prédéterminé et qui est reconnu comme présentant un risque vasculaire pouvant être réduit par thérapie d'abaissement de la pression artérielle à l'aide de médicaments antihypertenseurs ou
• la recommandation de ne pas administrer de médicaments antihypertenseurs à un sujet dont le taux déterminé de hGH et/ou de ses isoformes est inférieur audit seuil prédéterminé et qui est reconnu comme ne présentant pas de risque vasculaire pouvant être réduit par une thérapie d'abaissement de la pression artérielle à l'aide de médicaments antihypertenseurs.

17. Médicament antihypertenseur destiné à être utilisé dans une méthode de prévention d'un événement cardiovasculaire majeur et/ou d'une maladie cardiovasculaire chez un sujet dont le taux déterminé de hGH et/ou de ses isoformes choisies parmi le groupe comprenant l'isoforme 1 de hGH, l'isoforme 2 de hGH, l'isoforme 3 de hGH et l'isoforme 4 de hGH est supérieur audit seuil prédéterminé et ledit sujet est reconnu, selon les méthodes de l'une quelconque des revendications 1 à 16, comme présentant un risque vasculaire pouvant être réduit par thérapie d'abaissement de la pression artérielle à l'aide de médicaments antihypertenseurs, ledit événement cardiovasculaire étant choisi parmi le groupe comprenant l'infarctus du myocarde, l'insuffisance cardiaque aiguë, l'accident vasculaire cérébral et la mort cardiovasculaire.

18. Médicament antihypertenseur destiné à être utilisé dans une méthode de prévention d'un événement cardiovasculaire majeur et/ou d'une maladie cardiovasculaire chez un sujet dont le taux déterminé de hGH et/ou de ses isoformes choisies parmi le groupe comprenant l'isoforme 1 de hGH, l'isoforme 2 de hGH, l'isoforme 3 de hGH et l'isoforme 4 de hGH est supérieur audit seuil prédéterminé et ledit sujet est déterminé, selon les méthodes de l'une quelconque des revendications 1 à 16, comme présentant un risque vasculaire pouvant être réduit par thérapie d'abaissement de la pression sanguine à l'aide de médicaments antihypertenseurs, ledit sujet ne souffrant pas d'hypertension.

19. Méthode de surveillance de la réponse thérapeutique d'un sujet à risque vasculaire qui est traité à l'aide de médicaments antihypertenseurs, comprenant les étapes de :
• détermination du taux de hGH et/ou de ses isoformes choisies parmi le groupe comprenant l'isoforme 1 de hGH, l'isoforme 2 de hGH, l'isoforme 3 de hGH et l'isoforme 4 de hGH dans un échantillon de sang dudit sujet prélevé à un moment X1 avec [ML1] des médicaments antihypertenseurs et
• détermination du taux de hGH et/ou desdites isoformes de celle-ci dans un échantillon de sang dudit sujet prélevé à un moment X2 avec des [ML2]médicaments antihypertenseurs, et
• le moment X1 étant situé soit avant le début de la thérapie, soit après le début de la thérapie, mais dans tous les cas avant le moment X2, et
• le moment X2 étant situé après le début la thérapie et, dans tous les cas, après le moment X1
• comparaison du taux déterminé de hGH et/ou desdites isoformes de celle-ci dans ledit échantillon de sang prélevé au moment X1 avec le taux déterminé de hGH et/ou desdites isoformes de celle-ci dans ledit échantillon de sang prélevé au moment X2.
• un taux plus bas de hGH et/ou desdites isoformes de celle-ci dans ledit échantillon de sang prélevé au moment X2 par rapport au taux de hGH et/ou desdites isoformes de celle-ci dans ledit échantillon de sang prélevé au moment X1 indique une réponse thérapeutique.

20. Méthode permettant de surveiller la réponse thérapeutique d'un sujet à risque vasculaire qui est traité à l'aide de médicaments antihypertenseurs selon la revendication 19, le taux déterminé de hGH et/ou de ses isoformes choisis parmi le groupe qui comprend l'isoforme 1 de hGH, l'isoforme 2 de hGH, l'isoforme 3 de hGH et l'isoforme 4 de hGH dans ledit échantillon de sang prélevé au moment X1 étant supérieur à un seuil prédéterminé et le sujet ayant été déterminé comme présentant un risque vasculaire pouvant être réduit par une thérapie d'abaissement de la pression sanguine à l'aide de médicaments antihypertenseurs.

21. Méthode permettant de surveiller la réponse thérapeutique d'un sujet à risque vasculaire qui est traité à l'aide de médicaments antihypertenseurs selon la revendication 19 ou 20, le moment X1 étant situé avant le début de la thérapie.

22. Méthode permettant de surveiller la réponse thérapeutique d'un sujet à risque vasculaire qui est traité à l'aide de médicaments antihypertenseurs selon l'une quelconque des revendications 19 à 21, le moment X2 étant 12 mois, 24 mois ou 36 mois après le moment X1.
